# EUROPEAN PATENT APPLICATION

(11) **EP 4 628 505 A1**
(43) Date of publication of application: **08.10.2025**
(21) Application number: 23881999.9
(22) Date of filing: 30.10.2023
(51) Int. Cl.: C07K 16/28, C12N 15/13, C12N 15/63, C12N 5/07, A61K 39/395, A61P 35/00, G01N 33/574, G01N 33/68, G01N 33/577

(54) **NOVEL ANTI-CTLA4 ANTIBODY**

(30) Priority: 28.10.2022 WO PCT/CN2022/128253
(71) Applicant: Genor Biopharma Co., Ltd., Shanghai 201203 (CN)
(72) Inventor: DU, Qinglin, Shanghai 201203 (CN); LV, Yaxuan, Shanghai 201203 (CN); ZHOU, Tiantian, Shanghai 201203 (CN); PENG, Fei, Shanghai 201203 (CN); LI, Xueqin, Shanghai 201203 (CN); CAO, Huanhuan, Shanghai 201203 (CN); YANG, Xueyan, Shanghai 201203 (CN); DING, Qian, Shanghai 201203 (CN); TAN, Yongcong, Shanghai 201203 (CN); HAN, Shuhua, Shanghai 201203 (CN)
(74) Representative: Fritzsche, Thomas
(86) International application number: PCT/CN2023/127601
(87) International publication number: WO 2024/088424

(57) **Abstract**

The present disclosure provides antibodies, particularly heavy chain antibodies, and more particularly single domain antibodies, specifically binding to cytotoxic T lymphocyte-associated antigen-4 (CTLA4). Also disclosed are therapeutic uses of the antibodies.

## Description

### TECHNICAL FIELD

The present disclosure relates to antibodies and their uses. Specifically, the present disclosure relates to antibodies specifically binding to cytotoxic T lymphocyte-associated antigen-4 (CTLA4), in particular heavy chain antibodies, more particularly single domain antibodies; and therapeutic uses of the antibodies.

### BACKGROUND ART

As the first clinically targeted immune checkpoint receptor, cytotoxic T lymphocyte-associated antigen-4 (CTLA4, also known as CD152) is expressed in T cells. CTLA4 is constitutively expressed in regulatory T cells (Tregs) and upregulated in other T cells upon activation (Śledzińska et al., 2015, Jago et al., 2004). CTLA4 shares the same ligands as CD28, namely CD80/B7-1 and CD86/B7-2. Chikuma proposed that the mechanism of CTLA4 is to inhibit T cell activation by outcompeting CD28 in binding to CD80/B7-1 and CD86/B7-2 (Chikuma, 2017). CTLA4 is highly expressed in the tumor microenvironment (TME), especially on Tregs, which are considered to be key molecules for regulating Treg function and modulating anti-tumor immunity (Montler et al., 2016, Sutmuller et al., 2001). In addition, CTLA4 is constitutively expressed in Foxp3+Treg cells, inhibiting anti-tumor activity (Montler et al., 2016, Sutmuller et al., 2001).

So far, several mechanisms of action of therapeutic CTLA4 antibodies have been proposed, including activation of effector T cells by blocking the B7-CTLA4 pathway and depletion of Tregs through antibody-dependent cell-mediated cytotoxicity (ADCC), as well as antibody-dependent cellular phagocytosis (ADCP) (Arce Vargas et al., 2018, Du et al., 2018). However, the clinical importance of these mechanisms remains controversial (Du et al., 2018).

Ipilimumab (Yervoy^{®}) is the most well-known anti-CTLA4 antibody and was approved by FDA in 2011 for the treatment of advanced melanoma. Ipilimumab has shown effective cancer immunotherapy effects (CITE) in the clinic, both as a monotherapy (Hodi et al., 2010) and as part of a combination therapy with nivolumab (Larki et al., 2015). However, the above-mentioned CTLA4 therapies exhibit severe immunotherapy-related adverse events (irAEs) (Calabrese et al., 2018), especially when combined with nivolumab treatment, because systemic activation of T cells by blocking the B7-CTLA4 pathway leads to reduced patient tolerance to the antibody (Hodi, 2010, Bertrand et al., 2015).

Even so, CTLA4 remains an important immunotherapy target because of its ability to induce durable immune responses in cancer patients (Maio et al., 2015, Schadendorf et al., 2015). The main challenge in generating CTLA4 antibodies is to improve their safety and efficacy. Recently, Zhang et al. reported a novel CTLA4 antibody, HL32 (ONC-392) with a modified Fc, that uniquely targets CTLA4 to selectively eliminate tumor-infiltrating Tregs without affecting T cell activation in peripheral T cells (Zhang et al., 2019). Compared with other commercial or clinical-stage anti-CTLA4 antibodies, HL32 exhibits more robust CITEs but significantly reduced irAEs in preclinical models (Zhang et al., 2019, Du et al., 2018) and phase 1A/1B clinical trials (PRESERVE-001, NCT04140526). They proposed that clinically effective anti-CTLA4 mAbs would induce tumor rejection through a mechanism that is independent of checkpoint blockade but dependent on host Fc receptors (Du et al., 2018).

However, despite the above progress, there is still a need in the art for improved anti-CTLA-4 antibodies with increased response rate (RR) and reduced irAEs, as well as more effective immunotherapies.

### SUMMARY OF THE INVENTION

The present inventors have identified improved anti-CTLA4 antibodies with various blocking abilities against CD80/86 and retained Fc function, and thus providing improved response rates and reduced irAEs. As demonstrated in the Examples of the present application, the antibodies inhibited the binding of CD80/CD86 to CTLA4 to varying degrees, and also exhibited a potent ADCC response.

Thus, in one aspect, the present disclosure provides an antibody specifically binding to CTLA4, wherein the antibody comprises a heavy chain variable region comprising CDR1, CDR2, and CDR3, wherein the CDR1, CDR2, and CDR3 comprise an amino acid sequence that is at least 70%, 75%, 80%, 85%, 88%, 90%, 92%, 95%, 97%, 98%, 99%, or 100% identical to:
(1) SEQ ID NO: 1, SEQ ID NO: 2 and SEQ ID NO: 3, respectively;
(2) SEQ ID NO: 13, SEQ ID NO: 14 and SEQ ID NO: 15, respectively; or
(3) SEQ ID NO: 22, SEQ ID NO: 23 and SEQ ID NO: 24, respectively.

In some embodiments, the heavy chain variable region comprises CDR1, CDR2 and CDR3, wherein the CDR1 comprises or consists of the sequence as set forth in SEQ ID NO: 1, the CDR2 comprises or consists of the sequence as set forth in SEQ ID NO: 2, and the CDR3 comprises or consists of the sequence as set forth in SEQ ID NO: 3.

In some embodiments, the heavy chain variable region comprises an amino acid sequence that is at least 70%, 75%, 80%, 85%, 88%, 90%, 92%, 95%, 97%, 98%, 99% or 100% identical to SEQ ID NO: 4, SEQ ID NO: 7, SEQ ID NO: 10 or SEQ ID NO: 31.

In some embodiments, the heavy chain variable region comprises or consists of SEQ ID NO: 4.

In some embodiments, the heavy chain comprises an amino acid sequence that is at least 70%, 75%, 80%, 85%, 88%, 90%, 92%, 95%, 97%, 98%, 99%, or 100% identical to SEQ ID NO:5.

In some embodiments, the heavy chain variable region comprises or consists of SEQ ID NO: 7.

In some embodiments, the heavy chain comprises an amino acid sequence that is at least 70%, 75%, 80%, 85%, 88%, 90%, 92%, 95%, 97%, 98%, 99%, or 100% identical to SEQ ID NO: 8.

In some embodiments, the heavy chain variable region comprises or consists of SEQ ID NO: 10.

In some embodiments, the heavy chain comprises an amino acid sequence that is at least 70%, 75%, 80%, 85%, 88%, 90%, 92%, 95%, 97%, 98%, 99%, or 100% identical to SEQ ID NO: 11.

In some embodiments, the heavy chain variable region comprises or consists of SEQ ID NO: 31.

In some embodiments, the heavy chain comprises an amino acid sequence that is at least 70%, 75%, 80%, 85%, 88%, 90%, 92%, 95%, 97%, 98%, 99%, or 100% identical to SEQ ID NO: 32.

In some embodiments, the heavy chain variable region comprises CDR1, CDR2 and CDR3, wherein the CDR1 comprises or consists of the sequence as set forth in SEQ ID NO: 13, the CDR2 comprises or consists of the sequence as set forth in SEQ ID NO: 14, and the CDR3 comprises or consists of the sequence as set forth in SEQ ID NO: 15.

In some embodiments, the heavy chain variable region comprises an amino acid sequence that is at least 70%, 75%, 80%, 85%, 88%, 90%, 92%, 95%, 97%, 98%, 99% or 100% identical to SEQ ID NO: 16 or SEQ ID NO: 19.

In some embodiments, the heavy chain variable region comprises or consists of SEQ ID NO: 16.

In some embodiments, the heavy chain comprises an amino acid sequence that is at least 70%, 75%, 80%, 85%, 88%, 90%, 92%, 95%, 97%, 98%, 99%, or 100% identical to SEQ ID NO: 17.

In some embodiments, the heavy chain variable region comprises or consists of SEQ ID NO: 19.

In some embodiments, the heavy chain comprises an amino acid sequence that is at least 70%, 75%, 80%, 85%, 88%, 90%, 92%, 95%, 97%, 98%, 99%, or 100% identical to SEQ ID NO: 20.

In some embodiments, the heavy chain variable region comprises CDR1, CDR2 and CDR3, wherein the CDR1 comprises or consists of the sequence as set forth in SEQ ID NO: 22, the CDR2 comprises or consists of the sequence as set forth in SEQ ID NO: 23, and the CDR3 comprises or consists of the sequence as set forth in SEQ ID NO: 24.

In some embodiments, the heavy chain variable region comprises an amino acid sequence that is at least 70%, 75%, 80%, 85%, 88%, 90%, 92%, 95%, 97%, 98%, 99% or 100% identical to SEQ ID NO: 25 or SEQ ID NO: 28.

In some embodiments, the heavy chain variable region comprises or consists of SEQ ID NO: 25.

In some embodiments, the heavy chain comprises an amino acid sequence that is at least 70%, 75%, 80%, 85%, 88%, 90%, 92%, 95%, 97%, 98%, 99%, or 100% identical to SEQ ID NO: 26.

In some embodiments, the heavy chain variable region comprises or consists of SEQ ID NO: 28.

In some embodiments, the heavy chain comprises an amino acid sequence that is at least 70%, 75%, 80%, 85%, 88%, 90%, 92%, 95%, 97%, 98%, 99%, or 100% identical to SEQ ID NO: 29.

In some embodiments, the antibody or antigen-binding fragment thereof is selected from the group consisting of an intact antibody, a single-chain antibody (scFv), a heavy chain antibody, a single domain antibody, a Fab fragment, a Fab' fragment, F(ab')2, a Fv, a Fd fragment and a bispecific antibody (BsAb).

In some embodiments, the antibody or antigen-binding fragment thereof is a heavy chain-only antibody.

In some embodiments, the antibody or antigen-binding fragment thereof comprises or consists of two heavy chains.

In some embodiments, the antibody or antigen-binding fragment thereof is a single domain antibody.

In some embodiments, the antibody is a human antibody, a humanized antibody, or a chimeric antibody.

In some embodiments, the antibody or antigen-binding fragment thereof exhibits significant antibody-dependent cell-mediated cytotoxicity (ADCC).

In one aspect, the present disclosure provides a pharmaceutical composition comprising a therapeutically effective amount of the antibody or antigen-binding fragment thereof and a pharmaceutically acceptable carrier.

In one aspect, the present disclosure provides an isolated nucleic acid molecule encoding the antibody.

In some embodiments, the nucleic acid molecule comprises a nucleotide sequence that is at least 70%, 75%, 80%, 85%, 88%, 90%, 92%, 95%, 97%, 98%, 99% or 100% identical to SEQ ID NO: 6, SEQ ID NO: 9, SEQ ID NO: 12, SEQ ID NO: 18, SEQ ID NO: 21, SEQ ID NO: 27, SEQ ID NO: 30 or SEQ ID NO: 33.

In one aspect, the present disclosure provides an expression vector comprising the nucleic acid molecule.

In one aspect, the present disclosure provides a host cell, which expresses the antibody, and/or comprises the nucleic acid molecule or the expression vector.

In one aspect, the disclosure provides a method of treating a disease or condition in a subject in need thereof, comprising administering the antibody or pharmaceutical composition in a therapeutically effective amount, wherein the disease or condition is cancer or an autoimmune disease.

In some embodiments, the cancer is selected from the group consisting of colorectal cancer, colon cancer, renal cell carcinoma, breast cancer, epithelial squamous cell carcinoma, melanoma, myeloma, gastric cancer, brain cancer, lung cancer, pancreatic cancer, cervical cancer, ovarian cancer, liver cancer, bladder cancer, prostate cancer, testicular cancer, thyroid cancer, head and neck cancer, leukemia, and lymphoma.

In some embodiments, the antibody or the pharmaceutical composition is administered in combination with one or more additional chemotherapeutic agents, radiotherapeutic agents, cytokines, or other antibodies.

In one aspect, the present disclosure provides use of the antibody or the pharmaceutical composition in the preparation of a medicament for treating a disease or disorder, wherein the disease or disorder is cancer or an autoimmune disease.

In some embodiments, the cancer is selected from the group consisting of colorectal cancer, colon cancer, renal cell carcinoma, breast cancer, epithelial squamous cell carcinoma, melanoma, myeloma, gastric cancer, brain cancer, lung cancer, pancreatic cancer, cervical cancer, ovarian cancer, liver cancer, bladder cancer, prostate cancer, testicular cancer, thyroid cancer, head and neck cancer, leukemia, and lymphoma.

In some embodiments, the antibody or the pharmaceutical composition is for administration in combination with one or more additional chemotherapeutic agents, radiotherapeutic agents, cytokines or other antibodies.

In one aspect, the disclosure provides the antibody or the pharmaceutical composition for use in treating a disease or disorder, wherein the disease or disorder is cancer or an autoimmune disease.

In some embodiments, the cancer is selected from the group consisting of colorectal cancer, colon cancer, renal cell carcinoma, breast cancer, epithelial squamous cell carcinoma, melanoma, myeloma, gastric cancer, brain cancer, lung cancer, pancreatic cancer, cervical cancer, ovarian cancer, liver cancer, bladder cancer, prostate cancer, testicular cancer, thyroid cancer, head and neck cancer, leukemia, and lymphoma.

In some embodiments, the antibody or the pharmaceutical composition is for administration in combination with one or more additional chemotherapeutic agents, radiotherapeutic agents, cytokines, or other antibodies.

In one aspect, the present disclosure provides a kit comprising
a) the antibody or the pharmaceutical composition; and
b) instructions for use.

### BRIEF DESCRIPTION OF THE DRAWINGS

Figures 1A and 1B show FACS analysis of the binding specificity of anti-CTLA4 antibodies (GBD008-hS005, GBD008-hS005-3, GBD008-hS005-5, GBD008-S019, GBD008-hS019-4, GBD008-S004, GBD008-hS004-5 and GBD008-hS005-3-2) to cell surface human CTLA4 protein, where Ipi represents Ipilimumab.

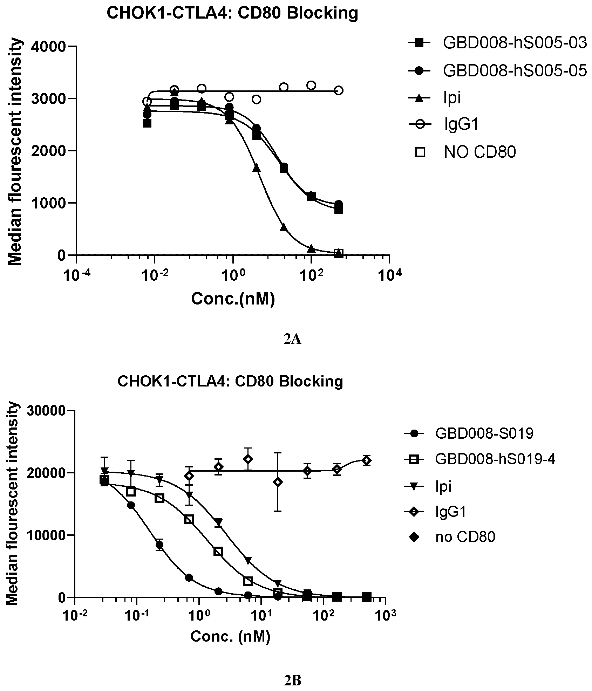

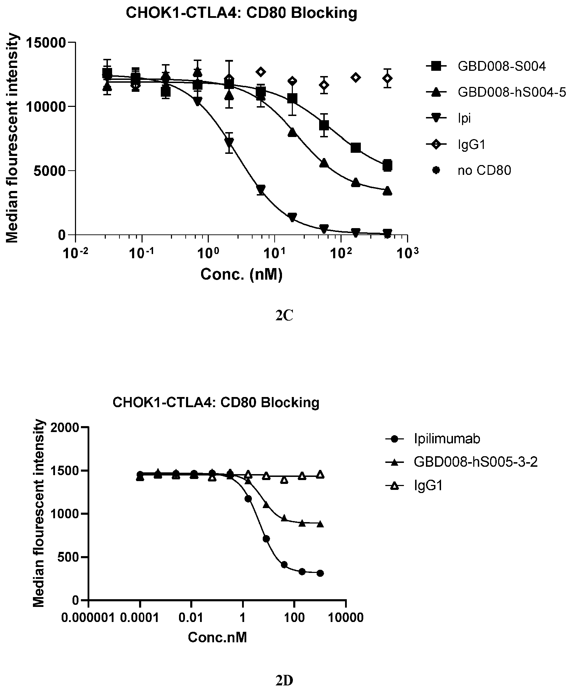

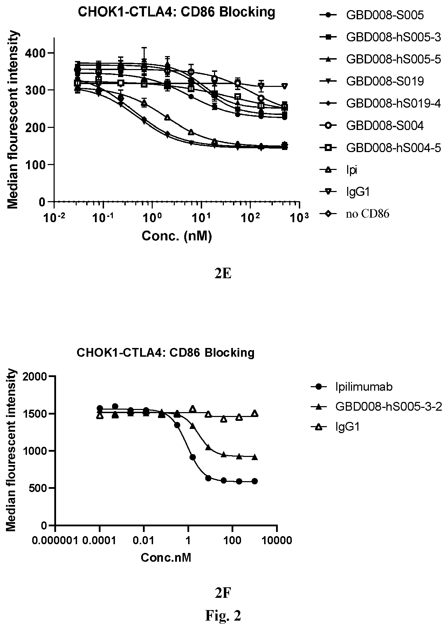

Figures 2A-2F show FACS analysis of the blockage of CD80 and CD86 binding to CTLA4 by anti-CTLA4 antibodies, where Ipi represents Ipilimumab. Figure 2A shows that anti-CTLA4 antibodies (GBD008-hS005-3 and GBD008-hS005-5) blocked CD80 binding to CTLA4; Figure 2B shows that anti-CTLA4 antibodies (GBD008-S019 and GBD008-hS019-4) blocked CD80 binding to CTLA4; Figure 2C shows that anti-CTLA4 antibodies (GBD008-S004 and GBD008-hS004-5) blocked CD80 binding to CTLA4; Figure 2D shows that anti-CTLA4 antibodies (GBD008-hS005-3-2) blocked CD80 binding to CTLA4; Figure 2E shows that anti-CTLA4 antibodies (GBD008-hS005, GBD008-hS005-3, GBD008-hS005-5, GBD008-S019, GBD008-hS019-4, GBD008-S004 and GBD008-hS004-5) blocked CD86 binding to CTLA4; Figure 2F shows that anti-CTLA4 antibody (GBD 008-hS005-3-2) blocked CD86 binding to CTLA4.
Figures 3A and 3B show the analysis of IL2 production by CTLA4 antibodies (GBD008-hS005, GBD008-hS005-3, GBD008-hS005-5, GBD008-S019, GBD008-hS019-4, GBD008-S004, GBD008-hS004-5, and GBD008-hS005-3-2) using SEB assay.
Figures 4A-4F show the binding ability of anti-CTLA4 antibodies (GBD008-hS005, GBD008-hS005-3, GBD008-hS005-5, GBD008-S019, GBD008-hS019-4, GBD008-S004, GBD008-hS004-5 and GBD008-hS005-3-2) to human CTLA4 (Figures 4A and 4B), cynomolgus monkey (*Macaca fascicularis*) CTLA4 (Figures 4C and 4D) and mouse CTLA4 (Figures 4E and 4F) (by ELISA).
Figure 5 shows the results of ADCC induced by anti-CTLA4 antibodies in an assay in which PBMCs were used as effector cells, Raji cells expressing full-length human CTLA4 were used as target cells (E: T=20:1), wherein Ipi represents Ipilimumab.
Figure 6 shows the in vivo anti-tumor efficacy by anti-CTLA4 antibodies in the MC38 colorectal tumor model. Figure 6A) Average tumor volume over time. Figure 6B) Average tumor volume and TGI (Tumor Growth Inhibition) at the end of the experiment.
Figures 7A-7C show the toxicity (arthritis) by anti-CTLA4 antibodies in hCTLA4/hPD1B alb/C mice. Figure 7A) Trend of weight change in mice. Figure 7B) Arthritis (paw and joint inflammation) scores in mice. Figure 7C) Incidence of arthritis in mice.

### DETAILED DESCRIPTION OF THE INVENTION

The present disclosure provides antibodies specifically binding to CTLA4. The disease or condition, including cancer and autoimmune diseases can be treated by administering a therapeutically effective amount of the anti-CTLA4 antibody of the present disclosure to a subject in need thereof. The binding of the antibody to T cells expressing CTLA4 induces strong antibody-dependent cell-mediated cytotoxicity against cells expressing tumor-associated antigens.

The antibodies disclosed herein provide various advantages, such as effective binding to CTLA4, potent induction of strong antibody-dependent cell-mediated cytotoxicity, and/or significantly reduced risk of adverse events (eg, toxicity).

In certain aspects, anti-CTLA4 antibodies in certain forms (e.g., heavy chain-only antibodies compared to typical full-length antibodies, i.e., heavy chain antibodies or single domain antibodies) bind to CTLA4 more potently, leading to higher efficacy and improved immunotherapy.

Unless otherwise defined, all scientific and technical terms used herein have the same meaning as commonly understood by one of ordinary skill in the art to which the present disclosure belongs. In the event of a conflict, the definitions in the present specification shall prevail.

The terminology used herein is for the purpose of describing particular embodiments only and is not intended to limit the invention. Although only certain exemplary materials and methods are described herein, many methods and materials similar or equivalent to those described herein can be used in the practice of the present disclosure.

Unless the context clearly indicates otherwise, the singular forms "a", "an" and "the" used herein are intended to include the plural forms as well. In addition, the open-ended expressions "including" and "comprising" are interpreted as also including structural components or method steps that are not described, but it should be noted that the open-ended expressions also cover the situation consisting of only the described components and method steps (i.e., covering the situation of the closed expression "consisting of...").

In general, the term "about" is used herein to modify a numerical value above and below the stated value by a variance of 5%.

As used throughout, ranges are used as shorthand for describing each and every value that is within the range. Any value within the range, such as an integer value, a value that increases by one-tenth (when the end value of the range is one decimal place) or a value that increases by one-hundredth (when the end value of the range is two decimal places), can be selected as the terminus of the range. For example, range 1-10 is used to describe all numerical values within the range, such as 1, 2, 3, 4, 5, 6, 7, 8... 9, 9.1, 9.2, 9.3, 9.4, 9.5, 9.6, 9.7, 9.8, 9.9 and 10 (values that increase by one-tenth), and includes all subranges, such as 1-1.5, 2.0-3.0, 4.0-5.0, 6.0-7.0, 8.0-9.0, etc.

The term "antibody" as used herein may include whole antibodies and any antigen binding fragments (i.e., "antigen-binding portions") or single chains thereof. An "antibody" refers, in one embodiment, to a glycoprotein comprising at least two heavy (H) chains and two light (L) chains inter-connected by disulfide bonds, or an antigen binding fragment thereof. Each heavy chain comprises a heavy chain variable region (abbreviated herein as VH) and a heavy chain constant region. In certain naturally occurring IgG, IgD and IgA antibodies, the heavy chain constant region comprises three domains, CH1, CH2 and CH3. In certain naturally occurring antibodies, each light chain comprises a light chain variable region (abbreviated herein as VL) and a light chain constant region. The light chain constant region comprises one domain, CL. The VH and VL regions can be further subdivided into regions of hypervariability, termed complementarity determining regions (CDR), interspersed with regions that are more conserved, termed framework regions (FR). Each VH and VL comprises three CDRs and four framework regions (FRs), arranged from amino-terminus to carboxy-terminus in the following order: FR1, CDR1, FR2, CDR2, FR3, CDR3, FR4. The variable regions of the heavy and light chains contain a binding domain that interacts with an antigen. The constant regions of the antibodies may mediate the binding of the immunoglobulin to host tissues or factors, including various cells of the immune system (e.g., effector cells) and the first component (C1q) of the classical complement system. Unless otherwise indicated, an immunoglobulin may be from any of the commonly known isotypes, including but not limited to IgA, secretory IgA, IgG and IgM. The IgG isotype is divided into subclasses in certain species: IgG1, IgG2, IgG3 and IgG4 in humans, and IgG1, IgG2a, IgG2b and IgG3 in mice. Immunoglobulins, e.g., human IgG1, exist in several allotypes, which differ from each other in at most a few amino acids.

Unless otherwise indicated, "antibody" may include, for example, intact antibodies, single-chain antibodies (scFv), heavy chain antibodies, single domain antibodies, Fab fragments, Fab' fragments, F(ab')2, Fv, Fd fragments, bispecific antibodies (BsAb), etc.; monoclonal and polyclonal antibodies; chimeric and humanized antibodies; human and non-human antibodies; wholly synthetic antibodies, etc.

As used herein, an "antigen binding fragment" of an antibody refers to one or more fragments of an antibody that retain the ability to specifically bind to an antigen bound by an intact antibody. "Antigen-binding fragments" are any proteinaceous structure that may exhibit binding affinity for a particular antigen. Antigen-binding fragments include those provided by any known technique, such as enzymatic cleavage, peptide synthesis, and recombinant techniques. Some antigen-binding fragments are composed of portions of intact antibodies that retain antigen-binding specificity of the parent antibody molecule. For example, antigen-binding fragments may comprise at least one variable region (either a heavy chain or light chain variable region) or one or more CDRs of an antibody known to bind to a particular antigen. Examples of suitable antigen-binding fragments include, without limitation diabodies and single-chain molecules as well as Fab, F(ab')2, Fc, Fabc, and Fv molecules, single chain (Sc) antibodies, individual antibody light chains, individual antibody heavy chains, chimeric fusions between antibody chains or CDRs and other proteins, protein scaffolds, heavy chain monomers or dimers, light chain monomers or dimers, dimers consisting of one heavy and one light chain, a monovalent fragment consisting of the VL, VH, CL and CH1 domains, or a monovalent antibody as described in WO2007059782, bivalent fragments comprising two Fab fragments linked by a disulfide bridge at the hinge region, a Fd fragment consisting essentially of the VH and CH1 domains; a Fv fragment consisting essentially of the VL and VH domains of a single arm of an antibody, a dAb fragment which consists essentially of a VH domain and also called domain antibodies; camelid or nanobodies; an isolated complementarity determining region (CDR), and the like. All antibody isotypes may be used to produce antigen-binding fragments. Additionally, antigen-binding fragments may include non-antibody proteinaceous frameworks that may successfully incorporate polypeptide segments in an orientation that confers affinity for a given antigen of interest, such as protein scaffolds. Antigen-binding fragments may be recombinantly produced or produced by enzymatic or chemical cleavage of intact antibodies.

As used herein, the term "heavy chain antibody" or "heavy chain-only antibody" refers to a single-chain antibody comprising only heavy chain(s). A "heavy chain antibody" lacks not only a light chain, but also a constant region CH1 region in the heavy chain of a conventional antibody. Compared to a heavy chain antibody, a "single domain antibody" as used herein lacks an Fc region.

As used herein, the term sequence "identity" refers to a relationship between two or more polynucleotide sequences or two or more polypeptide sequences. When a position in a sequence is occupied by the same nucleic acid base or amino acid residue in the corresponding position of the comparison sequence, the sequence is called "identical" at that position. The percentage sequence "identity" is calculated by determining the number of positions where the same nucleic acid base or amino acid residue appears in the two sequences to produce the number of "identical" positions. Then, the number of "identical" positions is divided by the total number of positions in the comparison window and multiplied by 100 to produce the percentage of sequence "identity". The percentage of "identity" is determined by comparing the two optimally aligned sequences over the comparison window. In order to optimally align the sequences for comparison, the portion of the polynucleotide or polypeptide sequence in the comparison window may include additions or deletions called gaps, while the reference sequence remains constant. The optimal alignment is an alignment that produces the maximum possible number of "identical" positions between the reference sequence and the comparison sequence even with gaps. For example, the percent "identity" between two sequences can be determined using a version of the program "BLAST 2 Sequences" available from the National Center for Biotechnology Information (NCBI), which includes the programs BLASTN (for nucleotide sequence comparisons) and BLASTP (for polypeptide sequence comparisons), which are based on the algorithm of Karlin and Altschul (Proc. Natl. Acad. Sci. USA 90(12):5873-5877, 1993).

A "humanized" antibody refers to an antibody in which some, most or all of the amino acids outside the CDR domains of a non-human antibody are replaced with corresponding amino acids derived from human immunoglobulins. In one embodiment of a humanized form of an antibody, some, most or all of the amino acids outside the CDR domains have been replaced with amino acids from human immunoglobulins, whereas some, most or all amino acids within one or more CDR regions are unchanged. Small additions, deletions, insertions, substitutions or modifications of amino acids are permissible as long as they do not abrogate the ability of the antibody to bind to a particular antigen. A "humanized" antibody retains an antigenic specificity similar to that of the original antibody.

A "chimeric antibody" refers to an antibody in which the variable regions are derived from one species and the constant regions are derived from another species, such as an antibody in which the variable regions are derived from a mouse antibody and the constant regions are derived from a human antibody. A"hybrid" antibody refers to an antibody with heavy and light chains of different types, e.g., a mouse (parent) heavy chain and a humanized light chain, or vice versa.

The term "monoclonal antibody," as used herein, refers to an antibody that displays a single binding specificity and affinity for a particular epitope or a composition of antibodies in which all antibodies display a single binding specificity and affinity for a particular epitope.

As used herein, "isotype" refers to the antibody class (e.g., IgGl, IgG2, IgG3, IgG4, IgM, IgAl, IgA2, IgD, and IgE antibody) that is encoded by the heavy chain constant domain genes.

As used herein, the terms "antibody-dependent cell-mediated cytotoxicity" and "ADCC" refer to a cell-mediated process in which nonspecific cytotoxic cells expressing FcγRs (e.g., monocytes, such as natural killer (NK) cells and macrophages) recognize bound antibodies (or other proteins capable of binding to FcγRs) on target cells and subsequently cause target cell lysis. In principle, any effector cell with an activated FcγR can be triggered to mediate ADCC. The predominant cells that mediate ADCC are NK cells, which express only FcγRIII, while monocytes can express FcγRI, FcγRII and FcγRIII depending on their activation, localization or differentiation state.

In one aspect, the present disclosure provides an antibody specifically binding to CTLA4, wherein the antibody comprises a heavy chain variable region, wherein the heavy chain variable region comprises CDR1, CDR2 and CDR3, wherein the CDR1, CDR2 and CDR3 comprise an amino acid sequence that is at least 70%, 75%, 80%, 85%, 88%, 90%, 92%, 95%, 97%, 98%, 99% or 100% identical to:
(1) SEQ ID NO: 1, SEQ ID NO: 2 and SEQ ID NO: 3, respectively;
(2) SEQ ID NO: 13, SEQ ID NO: 14 and SEQ ID NO: 15, respectively; or
(3) SEQ ID NO: 22, SEQ ID NO: 23 and SEQ ID NO: 24, respectively.

In some embodiments, the heavy chain variable region comprises CDR1, CDR2 and CDR3, wherein the CDR1 comprises or consists of the sequence as set forth in SEQ ID NO: 1, the CDR2 comprises or consists of the sequence as set forth in SEQ ID NO: 2, and the CDR3 comprises or consists of the sequence as set forth in SEQ ID NO: 3.

In some embodiments, the heavy chain variable region comprises an amino acid sequence that is at least 70%, 75%, 80%, 85%, 88%, 90%, 92%, 95%, 97%, 98%, 99% or 100% identical to SEQ ID NO: 4, SEQ ID NO: 7, SEQ ID NO: 10 or SEQ ID NO: 31.

In some embodiments, the heavy chain variable region comprises or consists of SEQ ID NO: 4.

In some embodiments, the heavy chain comprises an amino acid sequence that is at least 70%, 75%, 80%, 85%, 88%, 90%, 92%, 95%, 97%, 98%, 99%, or 100% identical to SEQ ID NO:5.

In some embodiments, the heavy chain variable region comprises or consists of SEQ ID NO: 7.

In some embodiments, the heavy chain comprises an amino acid sequence that is at least 70%, 75%, 80%, 85%, 88%, 90%, 92%, 95%, 97%, 98%, 99%, or 100% identical to SEQ ID NO: 8.

In some embodiments, the heavy chain variable region comprises or consists of SEQ ID NO: 10.

In some embodiments, the heavy chain comprises an amino acid sequence that is at least 70%, 75%, 80%, 85%, 88%, 90%, 92%, 95%, 97%, 98%, 99%, or 100% identical to SEQ ID NO: 11.

In some embodiments, the heavy chain variable region comprises or consists of SEQ ID NO: 31.

In some embodiments, the heavy chain comprises an amino acid sequence that is at least 70%, 75%, 80%, 85%, 88%, 90%, 92%, 95%, 97%, 98%, 99%, or 100% identical to SEQ ID NO: 32.

In some embodiments, the heavy chain variable region comprises CDR1, CDR2 and CDR3, wherein the CDR1 comprises or consists of the sequence as set forth in SEQ ID NO: 13, the CDR2 comprises or consists of the sequence as set forth in SEQ ID NO: 14, and the CDR3 comprises or consists of the sequence as set forth in SEQ ID NO: 15.

In some embodiments, the heavy chain variable region comprises an amino acid sequence that is at least 70%, 75%, 80%, 85%, 88%, 90%, 92%, 95%, 97%, 98%, 99% or 100% identical to SEQ ID NO: 16 or SEQ ID NO: 19.

In some embodiments, the heavy chain variable region comprises or consists of SEQ ID NO: 16.

In some embodiments, the heavy chain comprises an amino acid sequence that is at least 70%, 75%, 80%, 85%, 88%, 90%, 92%, 95%, 97%, 98%, 99%, or 100% identical to SEQ ID NO: 17.

In some embodiments, the heavy chain variable region comprises or consists of SEQ ID NO: 19.

In some embodiments, the heavy chain comprises an amino acid sequence that is at least 70%, 75%, 80%, 85%, 88%, 90%, 92%, 95%, 97%, 98%, 99%, or 100% identical to SEQ ID NO: 20.

In some embodiments, the heavy chain variable region comprises CDR1, CDR2 and CDR3, wherein the CDR1 comprises or consists of the sequence as set forth in SEQ ID NO: 22, the CDR2 comprises or consists of the sequence as set forth in SEQ ID NO: 23, and the CDR3 comprises or consists of the sequence as set forth in SEQ ID NO: 24.

In some embodiments, the heavy chain variable region comprises an amino acid sequence that is at least 70%, 75%, 80%, 85%, 88%, 90%, 92%, 95%, 97%, 98%, 99% or 100% identical to SEQ ID NO: 25 or SEQ ID NO: 28.

In some embodiments, the heavy chain variable region comprises or consists of SEQ ID NO: 25.

In some embodiments, the heavy chain comprises an amino acid sequence that is at least 70%, 75%, 80%, 85%, 88%, 90%, 92%, 95%, 97%, 98%, 99%, or 100% identical to SEQ ID NO: 26.

In some embodiments, the heavy chain variable region comprises or consists of SEQ ID NO: 28.

In some embodiments, the heavy chain comprises an amino acid sequence that is at least 70%, 75%, 80%, 85%, 88%, 90%, 92%, 95%, 97%, 98%, 99%, or 100% identical to SEQ ID NO: 29.

In some embodiments, the antibody or antigen-binding fragment thereof is selected from the group consisting of an intact antibody, a single chain antibody (scFv), a heavy chain antibody, a single domain antibody, a Fab fragment, a Fab' fragment, a F(ab')2, a Fv, a Fd fragment, and a bispecific antibody (BsAb).

In some embodiments, the antibodies of the present disclosure consist of only heavy chains. In some embodiments, the antibodies of the present disclosure comprise or consist of two heavy chains. In some embodiments, the antibodies of the present disclosure are single domain antibodies.

In some embodiments, the antibody is a human antibody, a humanized antibody, or a chimeric antibody.

In some embodiments, the antibody is a monoclonal antibody.

In some embodiments, the antibody is isolated.

In some embodiments, the antibody is an isolated monoclonal antibody.

Also provided are "conservative sequence modifications" to the antibody sequence provided herein, i.e. nucleotide and amino acid sequence modifications that do not abrogate the binding of the antibody encoded by the nucleotide sequence or containing the amino acid sequence, to the antigen. For example, modifications can be introduced by standard techniques known in the art, such as site-directed mutagenesis and PCR-mediated mutagenesis. Conservative sequence modifications include conservative amino acid substitutions, in which the amino acid residue is replaced with an amino acid residue having a similar side chain. Families of amino acid residues having similar side chains have been defined in the art. These families include amino acids with basic side chains (e.g., lysine, arginine, histidine), acidic side chains (e.g., aspartic acid, glutamic acid), uncharged polar side chains (e.g., glycine, asparagine, glutamine, serine, threonine, tyrosine, cysteine, tryptophan), nonpolar side chains (e.g., alanine, valine, leucine, isoleucine, proline, phenylalanine, methionine), beta-branched side chains (e.g., threonine, valine, isoleucine) and aromatic side chains (e.g., tyrosine, phenylalanine, tryptophan, histidine).

In some embodiments, the antibodies exhibit potent antibody-dependent cell-mediated cytotoxicity (ADCC).

In one aspect, the present disclosure provides a pharmaceutical composition comprising a therapeutically effective amount of the antibody and a pharmaceutically acceptable carrier, which may be an inert or physiologically active carrier. As used herein, the term "pharmaceutically acceptable carrier" includes any solvent, dispersion medium, coating, antibacterial agent, antifungal agent, etc. that is physiologically compatible. Examples of suitable carriers include water, saline, phosphate buffered saline, dextrose, glycerol, ethanol, etc., and any combination thereof.

In one aspect, the present disclosure provides an isolated nucleic acid molecule encoding the antibody.

In some embodiments, the nucleic acid molecule comprises a nucleotide sequence that is at least 70%, 75%, 80%, 85%, 88%, 90%, 92%, 95%, 97%, 98%, 99% or 100% identical to SEQ ID NO: 6, SEQ ID NO: 9, SEQ ID NO: 12, SEQ ID NO: 18, SEQ ID NO: 21, SEQ ID NO: 27, SEQ ID NO: 30 or SEQ ID NO: 33.

In some embodiments, the nucleic acid molecule comprises a nucleotide sequence that is at least 70%, 75%, 80%, 85%, 88%, 90%, 92%, 95%, 97%, 98%, 99% or 100% identical to SEQ ID NO: 6. In some embodiments, the nucleic acid molecule comprises the nucleotide sequence of SEQ ID NO: 6. In some embodiments, the nucleotide sequence of the nucleic acid molecule is set forth in SEQ ID NO: 6. In some embodiments, the nucleic acid molecule comprises a nucleotide sequence that is at least 70%, 75%, 80%, 85%, 88%, 90%, 92%, 95%, 97%, 98%, 99% or 100% identical to SEQ ID NO: 9. In some embodiments, the nucleic acid molecule comprises the nucleotide sequence of SEQ ID NO: 9. In some embodiments, the nucleotide sequence of the nucleic acid molecule is set forth in SEQ ID NO: 9. In some embodiments, the nucleic acid molecule comprises a nucleotide sequence that is at least 70%, 75%, 80%, 85%, 88%, 90%, 92%, 95%, 97%, 98%, 99% or 100% identical to SEQ ID NO: 12. In some embodiments, the nucleic acid molecule comprises the nucleotide sequence of SEQ ID NO: 12. In some embodiments, the nucleotide sequence of the nucleic acid molecule is set forth in SEQ ID NO: 12. In some embodiments, the nucleic acid molecule comprises a nucleotide sequence that is at least 70%, 75%, 80%, 85%, 88%, 90%, 92%, 95%, 97%, 98%, 99% or 100% identical to SEQ ID NO: 18. In some embodiments, the nucleic acid molecule comprises the nucleotide sequence of SEQ ID NO: 18. In some embodiments, the nucleotide sequence of the nucleic acid molecule is set forth in SEQ ID NO: 18. In some embodiments, the nucleic acid molecule comprises a nucleotide sequence that is at least 70%, 75%, 80%, 85%, 88%, 90%, 92%, 95%, 97%, 98%, 99% or 100% identical to SEQ ID NO: 21. In some embodiments, the nucleic acid molecule comprises the nucleotide sequence of SEQ ID NO: 21. In some embodiments, the nucleotide sequence of the nucleic acid molecule is set forth in SEQ ID NO: 21. In some embodiments, the nucleic acid molecule comprises a nucleotide sequence that is at least 70%, 75%, 80%, 85%, 88%, 90%, 92%, 95%, 97%, 98%, 99% or 100% identical to SEQ ID NO: 27. In some embodiments, the nucleic acid molecule comprises the nucleotide sequence of SEQ ID NO: 27. In some embodiments, the nucleotide sequence of the nucleic acid molecule is set forth in SEQ ID NO: 27. In some embodiments, the nucleic acid molecule comprises a nucleotide sequence that is at least 70%, 75%, 80%, 85%, 88%, 90%, 92%, 95%, 97%, 98%, 99% or 100% identical to SEQ ID NO: 30. In some embodiments, the nucleic acid molecule comprises the nucleotide sequence of SEQ ID NO: 30. In some embodiments, the nucleotide sequence of the nucleic acid molecule is set forth in SEQ ID NO: 30. In some embodiments, the nucleic acid molecule comprises a nucleotide sequence that is at least 70%, 75%, 80%, 85%, 88%, 90%, 92%, 95%, 97%, 98%, 99% or 100% identical to SEQ ID NO: 33. In some embodiments, the nucleic acid molecule comprises the nucleotide sequence of SEQ ID NO: 33. In some embodiments, the nucleotide sequence of the nucleic acid molecule is set forth in SEQ ID NO: 33.

In one aspect, the present disclosure provides an expression vector comprising the nucleic acid molecule.

In one aspect, the present disclosure provides a host cell, which expresses the antibody, and/or comprises the nucleic acid molecule or the expression vector.

In one aspect, the disclosure provides the antibody or the pharmaceutical composition for use in treating a disease or disorder, wherein the disease or disorder is cancer or an autoimmune disease.

In some embodiments, the cancer is selected from the group consisting of colorectal cancer, colon cancer, renal cell carcinoma, breast cancer, epithelial squamous cell carcinoma, melanoma, myeloma, gastric cancer, brain cancer, lung cancer, pancreatic cancer, cervical cancer, ovarian cancer, liver cancer, bladder cancer, prostate cancer, testicular cancer, thyroid cancer, head and neck cancer, leukemia, and lymphoma.

In some embodiments, the antibody or the pharmaceutical compositions is for administration in combination with one or more additional chemotherapeutic agents, radiotherapeutic agents, cytokines, or other antibodies.

In one aspect, the present disclosure provides use of the antibody or the pharmaceutical composition in the preparation of a medicament for treating a disease or disorder, wherein the disease or disorder is cancer or an autoimmune disease.

In some embodiments, the cancer is selected from the group consisting of colorectal cancer, colon cancer, renal cell carcinoma, breast cancer, epithelial squamous cell carcinoma, melanoma, myeloma, gastric cancer, brain cancer, lung cancer, pancreatic cancer, cervical cancer, ovarian cancer, liver cancer, bladder cancer, prostate cancer, testicular cancer, thyroid cancer, head and neck cancer, leukemia, and lymphoma.

In some embodiments, the antibody or the pharmaceutical compositions is for administration in combination with one or more additional chemotherapeutic agents, radiotherapeutic agents, cytokines, or other antibodies.

In one aspect, the disclosure provides a method of treating a disease or condition in a subject in need thereof, comprising administering the antibody or pharmaceutical composition in a therapeutically effective amount, wherein the disease or condition is cancer or an autoimmune disease.

In some embodiments, the cancer is selected from the group consisting of colorectal cancer, colon cancer, renal cell carcinoma, breast cancer, epithelial squamous cell carcinoma, melanoma, myeloma, gastric cancer, brain cancer, lung cancer, pancreatic cancer, cervical cancer, ovarian cancer, liver cancer, bladder cancer, prostate cancer, testicular cancer, thyroid cancer, head and neck cancer, leukemia, and lymphoma.

In some embodiments, the antibody or pharmaceutical composition is administered in combination with one or more additional chemotherapeutic agents, radiotherapeutic agents, cytokines, or other antibodies.

As used herein, "administering" refers to the introduction of a composition comprising a therapeutic agent to a subject, using any of the various methods and delivery systems known to those skilled in the art. Routes of administration for the pharmaceutical composition of the present disclosure include intravenous, intramuscular, subcutaneous, intraperitoneal, spinal or other parenteral routes of administration, for example by injection or infusion. The phrase "parenteral administration" as used herein means modes of administration other than enteral and topical administration, usually by injection, and includes, without limitation, intravenous, intramuscular, intraarterial, intrathecal, intralymphatic, intralesional, intracapsular, intraorbital, intracardiac, intradermal, intraperitoneal, transtracheal, subcutaneous, subcuticular, intraarticular, subcapsular, subarachnoid, intraspinal, epidural and intrasternal injection and infusion, as well as in vivo electroporation. In some embodiments, the composition is administered by non-parenteral routes, in some embodiments, by oral administration. Other non-parenteral routes include topical, epidermal or mucosal route of administration, for example, intranasal, vaginal, rectal, sublingual or topical. Administering can also be performed, for example, once, a plurality of times, and/or over one or more extended periods.

As used herein, the term "effective amount" refers to the amount of an active agent required to provide a therapeutic and/or prophylactic benefit to a subject.

The "subject in need thereof" refers to any mammal, such as but not limited to humans, horses, cows, cats, mice, rabbits, rats, goats, etc. Preferably, the mammal is a human.

In one aspect, the present disclosure provides a kit comprising
a) the antibody or the pharmaceutical composition; and
b) instructions for use.

**Table 1A. Sequence information**

| Sequence number | Specific sequence |
|---|---|
| SEQ ID NO: 1 | GRTASSNA |
| SEQ ID NO: 2 | ISATGGLT |
| SEQ ID NO: 3 | AVGRERWYLRHTENEYDY |
| SEQ ID NO: 4 | QVQLVESGGGLVEAGGSLRLSCATSGRTASSNAMAWFRQAPGKEREFVAAISATGG LTAYADSVKGRFTISRDNSKNAVYLQMNTLKPEDTAVYIGAVGRERWYLRHTENE YDYWGRGAQVTVSS |
| SEQ ID NO: 5 | QVQLVESGGGLVEAGGSLRLSCATSGRTASSNAMAWFRQAPGKEREFVAAISATGG LTAYADSVKGRFTISRDNSKNAVYLQMNTLKPEDTAVYIGAVGRERWYLRHTENE YDYWGRGAQVTVSS EPKSADKTHTCPPCPAPELLGGPSVFLFPPKPKDTLMISRTPEVTCVVVDVSHEDPEV KFNWYVDGVEVHNAKTKPREEQYNSTYRVVSVLTVLHQDWLNGKEYKCKVSNKA LPAPIEKTISKAKGQPREPQVYTLPPSREEMTKNQVSLTCLVKGFYPSDIAVEWESNG QPENNYKTTPPVLDSDGSFFLYSKLTVDKSRWQQGNVFSCSVMHEALHNHYTQKSL SLSPGK |
| SEQ ID NO: 6 | CAGGTCCAGCTCGTCGAGTCAGGCGGCGGCCTCGTGGAAGCCGGAGGAAGTTTG AGGCTCTCCTGCGCAACAAGCGGCAGGACCGCCAGCAGCAACGCCATGGCATG GTTTAGGCAGGCACCCGGCAAGGAGAGGGAGTTCGTGGCTGCTATCAGTGCCAC AGGAGGCTTGACAGCCTACGCCGACTCCGTGAAGGGAAGGTTCACAATCAGCAG AGACAACTCCAAGAACGCAGTTTACCTGCAGATGAACACACTGAAGCCCGAGG ACACCGCCGTGTATATTGGCGCCGTTGGCCGTGAAAGGTGGTACTTGAGGCACA CCGAGAACGAATACGACTACTGGGGCAGGGGCGCCCAGGTGACCGTCTCTTCC GAGCCCAAATCTGCTGACAAAACTCACACATGCCCACCGTGCCCAGCACCTGAA CTCCTGGGGGGACCGTCAGTCTTCCTCTTCCCCCCAAAACCCAAGGACACCCTCA TGATCTCCCGGACCCCCGAGGTCACATGCGTGGTGGACGTGAGCCACGAAG ACCCTGAGGTCAAGTTCAACTGGTACGTGGACGGCGTGGAGGTGCATAATGCCA AGACAAAGCCGCGGGAGGAGCAGTACAACAGCACGTACCGTGTGGTCAGCGTC CTCACCGTCCTGCACCAGGACTGGCTGAATGGCAAGGAGTACAAGTGCAAGGTC TCCAACAAAGCCCTCCCAGCCCCCATCGAGAAAACCATCTCCAAAGCCAAAGGG CAGCCCCGAGAACCACAGGTGTACACCCTGCCCCCATCCCGGGAGGAGATGACC AAGAACCAGGTCAGCCTGACCTGCCTGGTCAAAGGCTTCTATCCCAGCGACATC GCCGTGGAGTGGGAGAGCAATGGGCAGCCGGAGAACAACTACAAGACCACGCC TCCCGTGCTGGACTCCGACGGCTCCTTCTTCCTCTACAGCAAGCTCACCGTGGAC AAGAGCAGGTGGCAGCAGGGGAACGTCTTCTCATGCTCCGTGATGCATGAGGCT CTGCACAACCACTACACGCAGAAGAGCCTCTCCCTGTCTCCGGGTAAATGA |
| SEQ ID NO: 7 | EVQLVESGGGLVQPGGSLRLSCAASGRTASSNAMAWVRQAPGKEREFVSAISATGG LTAYADSVKGRFTISRDNSKNTLYLQMNSLRAEDTAVYYCAVGRERWYLRHTENE YDYWGQGTLVTVSS |
| SEQ ID NO: 8 | EVQLVESGGGLVQPGGSLRLSCAASGRTASSNAMAWVRQAPGKEREFVSAISATGG LTAYADSVKGRFTISRDNSKNTLYLQMNSLRAEDTAVYYCAVGRERWYLRHTENE YDYWGQGTLVTVSS EPKSADKTHTCPPCPAPELLGGPSVFLFPPKPKDTLMISRTPEVTCVVVDVSHEDPEV KFNWYVDGVEVHNAKTKPREEQYNSTYRVVSVLTVLHQDWLNGKEYKCKVSNKA LPAPIEKTISKAKGQPREPQVYTLPPSREEMTKNQVSLTCLVKGFYPSDIAVEWESNG QPENNYKTTPPVLDSDGSFFLYSKLTVDKSRWQQGNVFSCSVMIIEALIINIIYTQKSL SLSPGK |
| SEQ ID NO: 9 | GAGGTGCAGTTGGTGGAGAGCGGGGGGGGGCTGGTGCAGCCTGGAGGAAGTTT GAGGTTGAGCTGTGCCGCAAGCGGGAGGACAGCCAGTAGCAACGCAATGGCCT GGGTGAGGCAGGCACCCGGAAAGGAGAGGGAGTTCGTGAGTGCCATTTCCGCC ACCGGTGGACTGACCGCCTACGCCGACAGTGTGAAGGGGAGGTTCACCATCTCC AGGGACAACAGCAAGAACACCCTGTACCTGCAGATGAACAGCCTGAGGGCCGA GGACACAGCCGTGTACTACTGCGCAGTTGGCAGGGAGCGCTGGTACTTGAGGCA CACCGAGAACGAGTACGACTACTGGGGCCAGGGCACCCTGGTTACCGTGTCCTC C |
| | GAGCCCAAATCTGCTGACAAAACTCACACATGCCCACCGTGCCCAGCACCTGAA CTCCTGGGGGGACCGTCAGTCTTCCTCTTCCCCCCAAAACCCAAGGACACCCTCA TGATCTCCCGGACCCCCGAGGTCACATGCGTGGTGGACGTGAGCCACGAAG ACCCTGAGGTCAAGTTCAACTGGTACGTGGACGGCGTGGAGGTGCATAATGCCA AGACAAAGCCGCGGGAGGAGCAGTACAACAGCACGTACCGTGTGGTCAGCGTC CTCACCGTCCTGCACCAGGACTGGCTGAATGGCAAGGAGTACAAGTGCAAGGTC TCCAACAAAGCCCTCCCAGCCCCCATCGAGAAAACCATCTCCAAAGCCAAAGGG CAGCCCCGAGAACCACAGGTGTACACCCTGCCCCCATCCCGGGAGGAGATGACC AAGAACCAGGTCAGCCTGACCTGCCTGGTCAAAGGCTTCTATCCCAGCGACATC GCCGTGGAGTGGGAGAGCAATGGGCAGCCGGAGAACAACTACAAGACCACGCC TCCCGTGCTGGACTCCGACGGCTCCTTCTTCCTCTACAGCAAGCTCACCGTGGAC AAGAGCAGGTGGCAGCAGGGGAACGTCTTCTCATGCTCCGTGATGCATGAGGCT CTGCACAACCACTACACGCAGAAGAGCCTCTCCCTGTCTCCGGGTAAATGA |
| SEQ ID NO: 10 | EVQLVESGGGLVQPGGSLRLSCAASGRTASSNAMAWFRQAPGKEREFVSAISATGG LTAYADSVKGRFTISRDNSKNTLYLQMNSLRAEDTAVYIGAVGRERWYLRHTENEY DYWGQGTLVTVSS |
| SEQ ID NO: 11 | EVQLVESGGGLVQPGGSLRLSCAASGRTASSNAMAWFRQAPGKEREFVSAISATGG LTAYADSVKGRFTISRDNSKNTLYLQMNSLRAEDTAVYIGAVGRERWYLRHTENEY DYWGQGTLVTVSS EPKSADKTHTCPPCPAPELLGGPSVFLFPPKPKDTLMISRTPEVTCVVVDVSHEDPEV KFNWYVDGVEVHNAKTKPREEQYNSTYRVVSVLTVLHQDWLNGKEYKCKVSNKA LPAPIEKTISKAKGQPREPQVYTLPPSREEMTKNQVSLTCLVKGFYPSDIAVEWESNG QPENNYKTTPPVLDSDGSFFLYSKLTVDKSRWQQGNVFSCSVMHEALHNHYTQKSL SLSPGK |
| SEQ ID NO: 12 | GAGGTGCAGTTGGTGGAGAGCGGGGGGGGGCTGGTGCAGCCTGGAGGAAGTTT GAGGTTGAGCTGTGCCGCAAGCGGGAGGACAGCCAGTAGCAACGCAATGGCCT GGTTTAGGCAGGCACCCGGCAAGGAGAGGGAGTTCGTGAGTGCCATTTCCGCCA CCGGTGGACTGACAGCCTACGCCGACAGTGTGAAGGGCAGGTTCACCATCTCCA GGGACAACTCCAAGAACACCCTGTACCTGCAGATGAACAGCCTGAGGGCCGAG GACACCGCCGTGTACATCGGAGCCGTTGGCAGGGAGCGATGGTACTTGAGGCAC ACCGAGAACGAGTACGACTACTGGGGCCAGGGCACCCTGGTGACCGTGTCCAGC GAGCCCAAATCTGCTGACAAAACTCACACATGCCCACCGTGCCCAGCACCTGAA CTCCTGGGGGGACCGTCAGTCTTCCTCTTCCCCCCAAAACCCAAGGACACCCTCA TGATCTCCCGGACCCCCGAGGTCACATGCGTGGTGGTGGACGTGAGCCACGAAG ACCCTGAGGTCAAGTTCAACTGGTACGTGGACGGCGTGGAGGTGCATAATGCCA AGACAAAGCCGCGGGAGGAGCAGTACAACAGCACGTACCGTGTGGTCAGCGTC CTCACCGTCCTGCACCAGGACTGGCTGAATGGCAAGGAGTACAAGTGCAAGGTC TCCAACAAAGCCCTCCCAGCCCCCATCGAGAAAACCATCTCCAAAGCCAAAGGG CAGCCCCGAGAACCACAGGTGTACACCCTGCCCCCATCCCGGGAGGAGATGACC AAGAACCAGGTCAGCCTGACCTGCCTGGTCAAAGGCTTCTATCCCAGCGACATC GCCGTGGAGTGGGAGAGCAATGGGCAGCCGGAGAACAACTACAAGACCACGCC TCCCGTGCTGGACTCCGACGGCTCCTTCTTCCTCTACAGCAAGCTCACCGTGGAC AAGAGCAGGTGGCAGCAGGGGAACGTCTTCTCATGCTCCGTGATGCATGAGGCT CTGCACAACCACTACACGCAGAAGAGCCTCTCCCTGTCTCCGGGTAAATGA |
| SEQ ID NO: 13 | GFTFSSYTMA |
| SEQ ID NO: 14 | GISRSGGTTNYGDSVKG |
| SEQ ID NO: 15 | VSDY |
| SEQ ID NO: 16 | QVQLVESGGGLVQPGGSLTLSCAASGFTFSSYTMAWYRQAPGKEREVVAGISRSGG TTNYGDSVKGRFTISRDNAKNTVYLQMNSLKPEDTAVYYCRAVSDYWGQGTQVT VSS |
| SEQ ID NO: 17 | QVQLVESGGGLVQPGGSLTLSCAASGFTFSSYTMAWYRQAPGKEREVVAGISRSGG TTNYGDSVKGRFTISRDNAKNTVYLQMNSLKPEDTAVYYCRAVSDYWGQGTQVT VSS |
| | EPKSADKTHTCPPCPAPELLGGPSVFLFPPKPKDTLMISRTPEVTCVVVDVSHEDPEV KFNWYVDGVEVHNAKTKPREEQYNSTYRVVSVLTVLHQDWLNGKEYKCKVSNKA LPAPIEKTISKAKGQPREPQVYTLPPSREEMTKNQVSLTCLVKGFYPSDIAVEWESNG QPENNYKTTPPVLDSDGSFFLYSKLTVDKSRWQQGNVFSCSVMHEALHNHYTQKSL SLSPGK |
| SEQ ID NO: 18 | CAGGTGCAGCTGGTGGAAAGTGGCGGCGGCCTGGTGCAGCCCGGAGGAAGCCT GACACTGTCCTGCGCCGCCTCCGGTTTTACCTTCTCCTCCTACACAATGGCCTGG TACAGGCAGGCACCCGGCAAGGAGAGGGAGGTGGTGGCTGGCATTTCCAGAAG CGGAGGAACCACAAACTACGGGGATAGTGTGAAGGGCAGGTTTACCATTTCCAG GGACAACGCCAAGAACACCGTGTACCTGCAGATGAACAGCCTGAAGCCCGAGG ACACCGCAGTGTACTACTGCAGGGCCGTGAGCGACTACTGGGGCCAGGGGACCC AGGTGACCGTGAGCTCC |
| | GAGCCCAAATCTGCTGACAAAACTCACACATGCCCACCGTGCCCAGCACCTGAA CTCCTGGGGGGACCGTCAGTCTTCCTCTTCCCCCCAAAACCCAAGGACACCCTCA TGATCTCCCGGACCCCCGAGGTCACATGCGTGGTGGTGGACGTGAGCCACGAAG ACCCTGAGGTCAAGTTCAACTGGTACGTGGACGGCGTGGAGGTGCATAATGCCA AGACAAAGCCGCGGGAGGAGCAGTACAACAGCACGTACCGTGTGGTCAGCGTC CTCACCGTCCTGCACCAGGACTGGCTGAATGGCAAGGAGTACAAGTGCAAGGTC TCCAACAAAGCCCTCCCAGCCCCCATCGAGAAAACCATCTCCAAAGCCAAAGGG CAGCCCCGAGAACCACAGGTGTACACCCTGCCCCCATCCCGGGAGGAGATGACC AAGAACCAGGTCAGCCTGACCTGCCTGGTCAAAGGCTTCTATCCCAGCGACATC GCCGTGGAGTGGGAGAGCAATGGGCAGCCGGAGAACAACTACAAGACCACGCC TCCCGTGCTGGACTCCGACGGCTCCTTCTTCCTCTACAGCAAGCTCACCGTGGAC AAGAGCAGGTGGCAGCAGGGGAACGTCTTCTCATGCTCCGTGATGCATGAGGCT CTGCACAACCACTACACGCAGAAGAGCCTCTCCCTGTCTCCGGGTAAATGA |
| SEQ ID NO: 19 | EVQLLESGGGLVQPGGSLRLSCAASGFTFSSYTMAWYRQAPGKEREVVAGISRSGG TTNYGDSVKGRFTISRDNSKNTLYLQMNSLRAEDTAVYYCRAVSDYWGQGTLVTV SS |
| SEQ ID NO: 20 | EVQLLESGGGLVQPGGSLRLSCAASGFTFSSYTMAWYRQAPGKEREVVAGISRSGG TTNYGDSVKGRFTISRDNSKNTLYLQMNSLRAEDTAVYYCRAVSDYWGQGTLVTV SS |
| | EPKSADKTHTCPPCPAPELLGGPSVFLFPPKPKDTLMISRTPEVTCVVVDVSHEDPEV KFNWYVDGVEVHNAKTKPREEQYNSTYRVVSVLTVLHQDWLNGKEYKCKVSNKA LPAPIEKTISKAKGQPREPQVYTLPPSREEMTKNQVSLTCLVKGFYPSDIAVEWESNG QPENNYKTTPPVLDSDGSFFLYSKLTVDKSRWQQGNVFSCSVMHEALHNHYTQKSL SLSPGK |
| SEQ ID NO: 21 | GAAGTGCAACTACTAGAAAGTGGTGGTCTCGTGCAGCCTGGCGGATCTCTG AGACTGTCTTGTGCTGCTTCTGGCTTCACCTTCTCCTCCTACACCATGGCCTGGTA TCGCCAAGCCCCCGGAAAAGAGCGGGAAGTGGTGGCCGGCATCTCCAGATCCG GCGGCACCACCAACTACGGCGACTCCGTGAAGGGCAGATTTACCATCTCTCGGG ACAACTCCAAGAACACCCTGTACCTGCAGATGAATAGCCTGCGGGCCGAGGATA CAGCTGTGTACTACTGCAGAGCCGTGTCCGACTACTGGGGCCAGGGCACACTGG TCACCGTGTCTAGC GATAAGACACACACCTGCCCCCCTTGTCCTGCTCCCGAGCTGCTGGGCGGCCCTA GCGTGTTCCTGTTTCCACCCAAGCCTAAGGACACCCTGATGATCTCCCGGACACC CGAGGTGACCTGCGTGGTGGTGGACGTGTCTCACGAGGATCCTGAGGTGAAGTT CAACTGGTATGTGGATGGCGTGGAGGTGCACAATGCCAAGACCAAGCCCAGAG AGGAGCAGTACAACTCTACATATAGGGTGGTGAGCGTGCTGACCGTGCTGCACC AGGACTGGCTGAACGGCAAGGAGTATAAGTGCAAGGTGTCCAATAAGGCCCTG CCCGCCCCCATCGAGAAGACAATCAGCAAGGCCAAGGGCCAGCCTCGGGAGCC ACAGGTGTACACCCTGCCTCCATCCAGAGACGAGCTGACAAAGAACCAGGTGTC TCTGACATGTCTGGTGAAGGGCTTCTATCCTAGCGATATCGCCGTGGAGTGGGA GTCCAATGGCCAGCCAGAGAACAATTACAAGACCACACCCCCTGTGCTGGACTC CGATGGCTCCTTCTTTCTGTATTCCAAGCTGACCGTGGATAAGTCTCGGTGGCAG CAGGGCAACGTGTTCAGCTGTTCCGTGATGCACGAAGCCCTGCATAATCACTAT ACTCAGAAATCCCTGTCCCTGTCACCTGGAAAGTGATAA |
| SEQ ID NO: 22 | GRTFSSYDMG |
| SEQ ID NO: 23 | AISWGGVNTYYADSVKG |
| SEQ ID NO: 24 | GSAPYSSSRSSAQETYAQ |
| SEQ ID NO: 25 | EVQLVESGGGLVQAGGSLRLSCLASGRTFSSYDMGWFRQAPGKEREFVAAISWGG VNTYYADSVKGRFTISRDNAKNTVYLQMNSLKPEDTAVYYCARGSAPYSSSRSSAQ ETYAQWGQGTQVTVSS |
| SEQ ID NO: 26 | EVQLVESGGGLVQAGGSLRLSCLASGRTFSSYDMGWFRQAPGKEREFVAAISWGG VNTYYADSVKGRFTISRDNAKNTVYLQMNSLKPEDTAVYYCARGSAPYSSSRSSAQ ETYAQWGQGTQVTVSS |
| | EPKSADKTHTCPPCPAPELLGGPSVFLFPPKPKDTLMISRTPEVTCVVVDVSHEDPEV KFNWYVDGVEVHNAKTKPREEQYNSTYRVVSVLTVLHQDWLNGKEYKCKVSNKA LPAPIEKTISKAKGQPREPQVYTLPPSREEMTKNQVSLTCLVKGFYPSDIAVEWESNG QPENNYKTTPPVLDSDGSFFLYSKLTVDKSRWQQGNVFSCSVMHEALHNHYTQKSL SLSPGK |
| SEQ ID NO: 27 | GAGGTGCAGTTGGTGGAGAGCGGGGGGGGGCTGGTGCAGGCTGGAGGAAGTTT GAGGTTGAGCTGTTTGGCTTCTGGGAGGACCTTTAGCAGCTATGACATGGGATG GTTTAGGCAGGCCCCCGGAAAGGAGAGGGAGTTCGTGGCCGCCATTTCCTGGGG GGGGGTGAATACCTACTACGCTGACTCCGTGAAGGGACGATTCACAATCTCCCG GGACAACGCCAAGAACACAGTGTATCTGCAGATGAACAGCCTCAAGCCTGAAG ACACCGCTGTGTATTATTGCGCCCGGGGCTCCGCCCCCTATTCCTCTTCTAGGTC CTCCGCCCAGGAAACCTACGCCCAGTGGGGCCAGGGCACCCAGGTCACAGTGTC CTCC |
| | GAGCCCAAATCTGCTGACAAAACTCACACATGCCCACCGTGCCCAGCACCTGAA CTCCTGGGGGGACCGTCAGTCTTCCTCTTCCCCCCAAAACCCAAGGACACCCTCA TGATCTCCCGGACCCCCGAGGTCACATGCGTGGTGGTGGACGTGAGCCACGAAG ACCCTGAGGTCAAGTTCAACTGGTACGTGGACGGCGTGGAGGTGCATAATGCCA AGACAAAGCCGCGGGAGGAGCAGTACAACAGCACGTACCGTGTGGTCAGCGTC CTCACCGTCCTGCACCAGGACTGGCTGAATGGCAAGGAGTACAAGTGCAAGGTC TCCAACAAAGCCCTCCCAGCCCCCATCGAGAAAACCATCTCCAAAGCCAAAGGG CAGCCCCGAGAACCACAGGTGTACACCCTGCCCCCATCCCGGGAGGAGATGACC AAGAACCAGGTCAGCCTGACCTGCCTGGTCAAAGGCTTCTATCCCAGCGACATC GCCGTGGAGTGGGAGAGCAATGGGCAGCCGGAGAACAACTACAAGACCACGCC TCCCGTGCTGGACTCCGACGGCTCCTTCTTCCTCTACAGCAAGCTCACCGTGGAC AAGAGCAGGTGGCAGCAGGGGAACGTCTTCTCATGCTCCGTGATGCATGAGGCT CTGCACAACCACTACACGCAGAAGAGCCTCTCCCTGTCTCCGGGTAAATGATTCT AGA |
| SEQ ID NO: 28 | EVQLVESGGGLVQPGGSLRLSCAASGRTFSSYDMGWFRQAPGKEREFVAAISWGG VNTYYADSVKGRFTISRDNAKNTLYLQMNSLRAEDTAVYYCARGSAPYSSSRSSAQ ETYAQWGQGTLVTVSS |
| SEQ ID NO: 29 | EVQLVESGGGLVQPGGSLRLSCAASGRTFSSYDMGWFRQAPGKEREFVAAISWGG VNTYYADSVKGRFTISRDNAKNTLYLQMNSLRAEDTAVYYCARGSAPYSSSRSSAQ ETYAQWGQGTLVTVSS EPKSADKTHTCPPCPAPELLGGPSVFLFPPKPKDTLMISRTPEVTCVVVDVSHEDPEV KFNWYVDGVEVHNAKTKPREEQYNSTYRVVSVLTVLHQDWLNGKEYKCKVSNKA LPAPIEKTISKAKGQPREPQVYTLPPSREEMTKNQVSLTCLVKGFYPSDIAVEWESNG QPENNYKTTPPVLDSDGSFFLYSKLTVDKSRWQQGNVFSCSVMHEALHNHYTQKSL SLSPGK |
| SEQ ID NO: 30 | GAGGTGCAGCTGGTGGAGTCCGGAGGAGGACTGGTGCAGCCTGGAGGAAGCCT GAGGCTGTCCTGCGCTGCTTCCGGAAGGACCTTTAGTTCCTATGATATGGGGTGG TTCAGGCAGGCCCCTGGAAAAGAAAGGGAGTTCGTGGCTGCTATTTCCTGGGGG GGCGTGAACACCTACTACGCCGACTCCGTGAAGGGCAGGTTCACCATCTCCAGG GACAACGCCAAGAACACCCTGTACCTGCAGATGAACTCCCTGAGGGCCGAGGAC ACCGCCGTGTACTACTGCGCCAGGGGCTCCGCCCCCTACTCCTCCTCCAGGTCCT CCGCCCAGGAGACATACGCCCAGTGGGGCCAGGGCACCCTGGTGACCGTGTCCT CCGAGCCCAAATCTGCTGACAAAACTCACACATGCCCACCGTGCCCAGCACCTG AACTCCTGGGGGGACCGTCAGTCTTCCTCTTCCCCCCAAAACCCAAGGACACCCT CATGATCTCCCGGACCCCCGAGGTCACATGCGTGGTGGTGGACGTGAGCCACGA AGACCCTGAGGTCAAGTTCAACTGGTACGTGGACGGCGTGGAGGTGCATAATGC CAAGACAAAGCCGCGGGAGGAGCAGTACAACAGCACGTACCGTGTGGTCAGCG TCCTCACCGTCCTGCACCAGGACTGGCTGAATGGCAAGGAGTACAAGTGCAAGG TCTCCAACAAAGCCCTCCCAGCCCCCATCGAGAAAACCATCTCCAAAGCCAAAG GGCAGCCCCGAGAACCACAGGTGTACACCCTGCCCCCATCCCGGGAGGAGATGA CCAAGAACCAGGTCAGCCTGACCTGCCTGGTCAAAGGCTTCTATCCCAGCGACA TCGCCGTGGAGTGGGAGAGCAATGGGCAGCCGGAGAACAACTACAAGACCACG CCTCCCGTGCTGGACTCCGACGGCTCCTTCTTCCTCTACAGCAAGCTCACCGTGG ACAAGAGCAGGTGGCAGCAGGGGAACGTCTTCTCATGCTCCGTGATGCATGAGG CTCTGCACAACCACTACACGCAGAAGAGCCTCTCCCTGTCTCCGGGTAAATGA |
| SEQ ID NO: 31 | EVQLVESGGGLVQPGGSLRLSCAASGRTASSNAMAWFRQAPGKEREFVSAISATGG LTAYADSVKGRFTISRDNSKNTLYLQMNSLRAEDTAVYYCAVGRERWYLRHTENE YDYWGQGTLVTVSS |
| SEQ ID NO: 32 | EVQLVESGGGLVQPGGSLRLSCAASGRTASSNAMAWFRQAPGKEREFVSAISATGG LTAYADSVKGRFTISRDNSKNTLYLQMNSLRAEDTAVYYCAVGRERWYLRHTENE YDYWGQGTLVTVSS EPKSADKTHTCPPCPAPELLGGPSVFLFPPKPKDTLMISRTPEVTCVVVDVSHEDPEV KFNWYVDGVEVHNAKTKPREEQYNSTYRVVSVLTVLHQDWLNGKEYKCKVSNKA LPAPIEKTISKAKGQPREPQVYTLPPSRDELTKNQVSLTCLVKGFYPSDIAVEWESNG QPENNYKTTPPVLDSDGSFFLYSKLTVDKSRWQQGNVFSCSVMHEALHNHYTQKSL SLSPGK |
| SEQ ID NO: 33 | GAAGTGCAGCTGGTGGAGAGCGGAGGAGGATTGGTGCAGCCTGGAGGAAGCCT GAGGCTGAGTTGTGCCGCTAGTGGGAGGACAGCAAGTAGTAACGCAATGGCAT GGTTTAGGCAGGCCCCCGGAAAAGAGAGGGAGTTCGTGAGCGCCATTTCCGCCA CCGGAGGATTGACAGCCTACGCTGATTCCGTGAAGGGCAGGTTCACCATCAGCA GGGACAACTCCAAGAACACACTGTACCTGCAGATGAACAGCCTGAGGGCCGAG GACACAGCCGTTTACTACTGCGCCGTGGGCAGGGAAAGGTGGTACTTGAGGCAC ACCGAGAACGAGTACGACTACTGGGGCCAGGGCACCTTGGTGACAGTCTCTTCC GAGCCTAAAAGCGCCGACAAAACTCACACATGCCCACCGTGCCCAGCACCTGAA CTCCTGGGGGGACCGTCAGTCTTCCTCTTCCCCCCAAAACCCAAGGACACCCTCA TGATCTCCCGGACCCCCGAGGTCACATGCGTGGTGGTGGACGTGAGCCACGAAG ACCCTGAGGTCAAGTTCAACTGGTACGTGGACGGCGTGGAGGTGCATAATGCCA AGACAAAGCCGCGGGAGGAGCAGTACAACAGCACGTACCGTGTGGTCAGCGTC CTCACCGTCCTGCACCAGGACTGGCTGAATGGCAAGGAGTACAAGTGCAAGGTC TCCAACAAAGCCCTCCCAGCCCCCATCGAGAAAACCATCTCCAAAGCCAAAGGG CAGCCCCGAGAACCACAGGTGTACACCCTGCCCCCATCCCGGGACGAGCTGACC AAGAACCAGGTCAGCCTGACCTGCCTGGTCAAAGGCTTCTATCCCAGCGACATC GCCGTGGAGTGGGAGAGCAATGGGCAGCCGGAGAACAACTACAAGACCACGCC TCCCGTGCTGGACTCCGACGGCTCCTTCTTCCTCTACAGCAAGCTCACCGTGGAC AAGAGCAGGTGGCAGCAGGGGAACGTCTTCTCATGCTCCGTGATGCATGAGGCT CTGCACAACCACTACACGCAGAAGAGCCTCTCCCTGTCTCCGGGTAAATGA |

**Table 1B. Sequence description**

| Sequence number | Sequence Description |
|---|---|
| SEQ ID NO: 1 | Heavy chain CDR1 of antibody GBD008-hS005 |
| SEQ ID NO: 2 | Heavy chain CDR2 of antibody GBD008-hS005 |
| SEQ ID NO: 3 | Heavy chain CDR3 of antibody GBD008-hS005 |
| SEQ ID NO: 4 | Heavy chain variable region of antibody GBD008-hS005 |
| SEQ ID NO: 5 | Heavy chain of antibody GBD008-hS005 |
| SEQ ID NO: 6 | Heavy chain nucleotide sequence of antibody GBD008-hS005 |
| SEQ ID NO: 1 | Heavy chain CDR1 of antibody GBD008-hS005-3 |
| SEQ ID NO: 2 | Heavy chain CDR2 of antibody GBD008-hS005-3 |
| SEQ ID NO: 3 | Heavy chain CDR3 of antibody GBD008-hS005-3 |
| SEQ ID NO: 7 | Heavy chain variable region of antibody GBD008-hS005-3 |
| SEQ ID NO: 8 | Heavy chain of antibody GBD008-hS005-3 |
| SEQ ID NO: 9 | Heavy chain nucleotide sequence of antibody GBD008-hS005-3 |
| SEQ ID NO: 1 | Heavy chain CDR1 of antibody GBD008-hS005-5 |
| SEQ ID NO: 2 | Heavy chain CDR2 of antibody GBD008-hS005-5 |
| SEQ ID NO: 3 | Heavy chain CDR3 of antibody GBD008-hS005-5 |
| SEQ ID NO: 10 | Heavy chain variable region of antibody GBD008-hS005-5 |
| SEQ ID NO: 11 | Heavy chain of antibody GBD008-hS005-5 |
| SEQ ID NO: 12 | Heavy chain nucleotide sequence of antibody GBD008-hS005-5 |
| SEQ ID NO: 13 | Heavy chain CDR1 of antibody GBD008-5019 |
| SEQ ID NO: 14 | Heavy chain CDR2 of antibody GBD008-5019 |
| SEQ ID NO: 15 | Heavy chain CDR3 of antibody GBD008-5019 |
| SEQ ID NO: 16 | Heavy chain variable region of antibody GBD008-S019 |
| SEQ ID NO: 17 | Heavy chain of antibody GBD008-5019 |
| SEQ ID NO: 18 | Heavy chain nucleotide sequence of antibody GBD008-S019 |
| SEQ ID NO: 13 | Heavy chain CDR1 of antibody GBD008-hS019-4 |
| SEQ ID NO: 14 | Heavy chain CDR2 of antibody GBD008-hS019-4 |
| SEQ ID NO: 15 | Heavy chain CDR3 of antibody GBD008-hS019-4 |
| SEQ ID NO: 19 | Heavy chain variable region of antibody GBD008-hS019-4 |
| SEQ ID NO: 20 | Heavy chain of antibody GBD008-hS019-4 |
| SEQ ID NO: 21 | Heavy chain nucleotide sequence of antibody GBD008-hS019-4 |
| SEQ ID NO: 22 | Heavy chain CDR1 of antibody GBD008-S004 |
| SEQ ID NO: 23 | Heavy chain CDR2 of antibody GBD008-S004 |
| SEQ ID NO: 24 | Heavy chain CDR3 of antibody GBD008-S004 |
| SEQ ID NO: 25 | Heavy chain variable region of antibody GBD008-S004 |
| SEQ ID NO: 26 | Heavy chain of antibody GBD008-S004 |
| SEQ ID NO: 27 | Heavy chain nucleotide sequence of antibody GBD008-S004 |
| SEQ ID NO: 22 | Heavy chain CDR1 of antibody GBD008-hS004-5 |
| SEQ ID NO: 23 | Heavy chain CDR2 of antibody GBD008-hS004-5 |
| SEQ ID NO: 24 | Heavy chain CDR3 of antibody GBD008-hS004-5 |
| SEQ ID NO: 28 | Heavy chain variable region of antibody GBD008-hS004-5 |
| SEQ ID NO: 29 | Heavy chain of antibody GBD008-hS004-5 |
| SEQ ID NO: 30 | Heavy chain nucleotide sequence of antibody GBD008-hS004-5 |
| SEQ ID NO: 1 | Heavy chain CDR1 of antibody GBD008- hS00 5-3-2 |
| SEQ ID NO: 2 | Heavy chain CDR2 of antibody GBD008- hS00 5-3-2 |
| SEQ ID NO: 3 | Heavy chain CDR3 of antibody GBD008- hS00 5-3-2 |
| SEQ ID NO: 31 | Heavy chain variable region of antibody GBD008-hS00 5-3-2 |
| SEQ ID NO: 32 | Heavy chain of antibody GBD008-hS00 5-3-2 |
| SEQ ID NO: 33 | Heavy chain nucleotide sequence of antibody GBD008- hS00 5-3-2 |

The sequences of exemplary antibodies disclosed herein are shown in Table 2 below:

**Table 2: Full length sequences of exemplary antibodies**

| Antibody Designation | Sequence ID |
|---|---|
| GBD008-hS005 | SEQ ID NO: 5 |
| GBD008-hS005-3 | SEQ ID NO: 8 |
| GBD008-hS005-5 | SEQ ID NO: 11 |
| GBD008-S019 | SEQ ID NO: 17 |
| GBD008-hS019-4 | SEQ ID NO: 20 |
| GBD008-S004 | SEQ ID NO: 26 |
| GBD008-hS004-5 | SEQ ID NO: 29 |
| GBD 008-hS005-3-2 | SEQ ID NO: 32 |

The invention is further described with reference to the following non-limiting examples.

### Examples

The following exemplarily describes the development, characterization and in vivo anti-tumor efficacy of the above eight antibodies: GBD008-hS005, GBD008-hS005-3, GBD008-hS005-5, GBD008-S019, GBD008-hS019-4, GBD008-S004, GBD008-hS004-5 and GBD008-hS005-3-2.

### Example 1. Preparation of anti-CTLA4 antibodies

Camelids, such as camels or alpacas, are ideal animal models for producing antibodies containing only one heavy chain variable region (VHH) and two conventional CH2 and CH3 regions. VHH antibodies naturally lack light chains, but retain structural stability and effective antigen binding properties. Methods for obtaining antigen- or epitope-specific VHH domains have been previously described, for example in WO2006/040153 and WO2006/122786; R. van der Linden et al., Journal of Immunological Methods, 240 (2000) 185-195; Li et al., J Biol Chem., 287 (2012) 13713-13721; Deffar et al., African Journal of Biotechnology Vol. 8 (12), pp. 2645-2652, 17 Jun. 2009 and WO94/04678. The anti-CTLA4 VHH antibody sequence was obtained by immunizing alpacas with human CTLA4/CD153 protein (Acro Biosystem, Catalog #CT4-H5229). The VHH sequence obtained from alpacas was sequence optimized by replacing the amino acids in the original VHH sequence with the conventional amino acids that appear in the VH domain of human antibodies.

### Example 2. FACS analysis of the binding specificity of anti-CTLA4 antibodies to human CTLA4 protein on cell surface

CHOK1-CTLA4 cells (1x10⁵/well) were washed twice in FACS buffer (PBS + 2% BSA), resuspended in 100 µl FACS buffer containing serial dilutions (1:5) of anti-CTLA4 mAb, and incubated for 1 hour at 4°C. The cells were then washed twice in FACS buffer and the bound antibodies were detected by incubation with APC anti-human IgG Fc (Biolegend, Catalog #410712) for 1 hour at 4°C. Afterwards, cells were washed twice in FACS buffer and then collected and analyzed using BDL SR Fortessa (BD Biosciences).

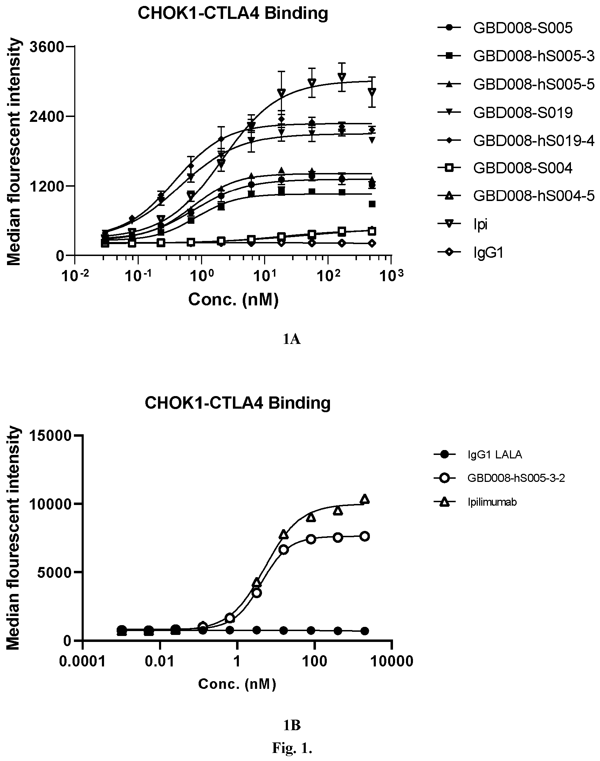

The results from Figures 1 A and B showed that GBD008-S004, GBD008-hS005, GBD008-S019, humanized GBD008-hS005-3, GBD008-hS005-5, GBD008-hS019-4, GBD008-hS004-5 and GBD008-hS005-3-2 antibodies all demonstrated specific binding to CTLA4 protein. In particular, the trend of the mean fluorescence intensity (MFI) of GBD008-S019 and GBD008-hS019-4 was similar to that of Ipilimumab, indicating that their binding specificity was comparable to that of Ipilimumab. The trend of the mean fluorescence intensity (MFI) of GBD008-S005, GBD008-hS005-3, GBD008-hS005-3-2 and GBD008-hS005-5 came second, and the trend of the mean fluorescence intensity (MFI) of GBD008-S004 and GBD008-hS004-5 (binding to CTLA4) was weaker.

### Example 3. FACS analysis of anti-CTLA4 antibodies blocking CD80 and CD86 binding to CTLA4

CHOK1-CTLA4 cells (1x10⁵/well) were washed twice in FACS buffer (PBS + 2% BSA), resuspended in 100 µl FACS buffer containing serial dilutions (1:5) of anti-CTLA4 mAb and CD80 Biotinylated tag (SinoBiological, Catlog #10698-H49H-B) or CD86 Biotinylated tag (SinoBiological, Catlog #10699-H08H-B), and incubated for 0.5 hour at 4°C. The cells were then washed twice in FACS buffer and the bound antibodies were detected by incubation with Streptavidi-Alexa Flour 488 (Invitrogen, Catlog #S32354) for 0.5 hour at 4°C. Afterwards, the cells were washed twice in FACS buffer and then collected and analyzed using BDL SR Fortessa (BD Bio sciences).

The results from Figures 2 A-F showed that the anti-CTLA4 antibodies: GBD008-S004, GBD008-hS005, GBD008-S019, humanized GBD008-hS005-3, GBD008-hS005-5, GBD008-hS005-3-2, GBD008-hS019-4, and GBD008-hS004-5 antibodies partially inhibited the binding of CD80 or CD86 to CTLA4. In particular, the trend of the mean fluorescence intensity (MFI) of GBD008-S019 and GBD008- hS019-4 was almost the same as that of Ipilimumab, indicating that their blocking ability was comparable to that of Ipilimumab. The trend of the mean fluorescence intensity (MFI) of GBD008-S005, GBD008-hS005-3, GBD008-hS005-3-2 and GBD008-hS005-5 came second, indicating that their blocking abilities were second, and the trend of the mean fluorescence intensity (MFI) of GBD008-S004 and GBD008- hS004-5 (binding to CTLA4) were weaker, indicating that their blocking abilities were weaker.

### Example 4. Affinity of CTLA-4 Antibody Fc Fusion Protein for CTLA-4 (Octet Method)

The binding kinetics of CTLA-4 antibody Fc fusion protein and recombinant human CTLA-4 were qualitatively and quantitatively analyzed by the Octet method for protein interaction (Octect RH-16, Sartorius). The association rate (kₒₙ) and dissociation rate (k_{off}) were calculated using a simple one-to-one binding model (Octet evaluation software version 12.2). The equilibrium dissociation constant (k_{D)} was calculated as the ratio of k_{off}/kₒₙ. The results are shown in Table 3. The results in Table 3 show that all CTLA4 antibody Fc fusion proteins bind to the CTLA4 antigen with strong affinity.

**Table 3. Results of affinity of CTLA4 antibody Fc fusion protein for CTLA4**

| **Antibody** | **K _{D}(M)** | **k ₒₙ (1/Ms)** | **k o _{ff} (1/s)** |
|---|---|---|---|
| Ipilimumab | 2.607E-10 | 2.204E05 | 5.746E-05 |
| GBD008-hS005 | 2.693E-10 | 2.153E05 | 5.798E-05 |
| GBD008-hS005-3 | 5.236E-10 | 1.892E05 | 9.906E-05 |
| GBD008-hS005-5 | 1.553E-10 | 1.971E05 | 3.061E-05 |
| GBD008-S019 | 1.654E-10 | 2.630E05 | 4.351E-05 |
| GBD008-hS019-4 | 2.363E-10 | 3.018E05 | 7.131E-05 |
| GBD008-S004 | 2.888E-08 | 1.734E05 | 5.008E-03 |
| GBD008-hS004-5 | 2.787E-08 | 2.406E05 | 6.706E-03 |
| GBD008-hS005-3-2 | 5.305E-10 | 2.140E05 | 1.136E-04 |

### Example 5. SEB assay for analyzing IL2 production by using CTLA4 antibody

Serially diluted antibodies and staphylococcal enterotoxin B (SEB) (Sigma-Aldrich, Catlog #S4881) were added to effector human peripheral blood mononuclear cells (1x10⁵/well), which were obtained from individual donors (Milestone^{®} Biotechnologies) and cultured in 10% FBS (Gibco, Catlog #10099-141) in RPMI 1640 medium (Gibco, Catlog #A10491-01). The cells were cultured at 37°C for 4 days, and then the sample supernatants were collected and mixed with premixed IL2-HTRF antibody (PerkinElmer, Catlog #62HIL02PET). The sample plate was sealed and incubated at room temperature for 3 hours. Afterwards, the plate seal was removed and the samples were read on an EnVision 2105 multi-mode plate reader (PerkinElmer).

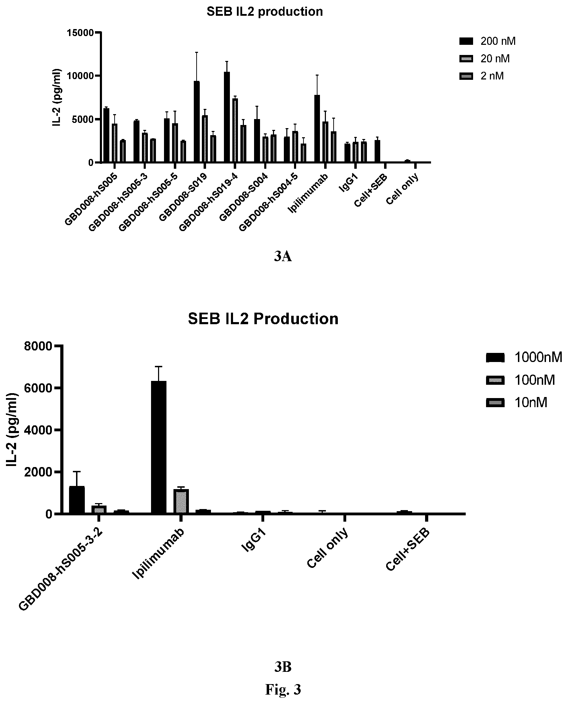

The results from Figures 3 A and B showed that GBD008-S004, GBD008-hS005, GBD008-S019, humanized GBD008-hS005-3, GBD008-hS005-3-2, GBD008-hS005-5, GBD008-hS019-4 and GBD008-hS004-5 antibodies all significantly promoted IL2 production in the effector cells in a concentration-dependent manner, among which the production of IL2 by GBD008-S019 and GBD008-hS019-4 was comparable to that of Ipilimumab. The production of IL2 by GBD008-S004, GBD008-hS005, and humanized GBD008-hS005-3, GBD008-hS005-3-2, and GBD008-hS005-5 came second. GBD008-hS004-5 (binding to CTLA4) had lower IL2 production. These results suggested that the antibodies of the present disclosure have the potential to treat cancer or autoimmune diseases.

### Example 6. Binding ability of anti-CTLA4 antibodies to cynomolgus monkey CTLA4 and mouse CTLA4 (by ELISA)

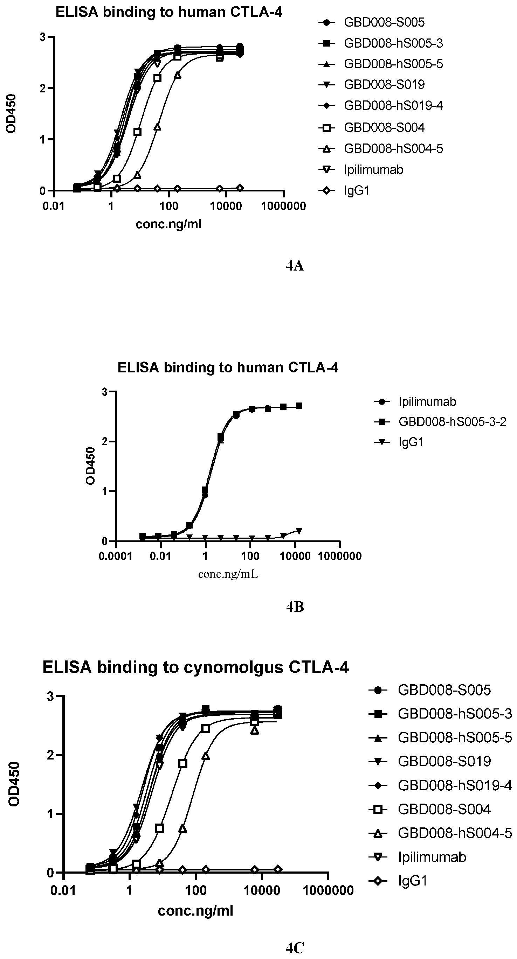

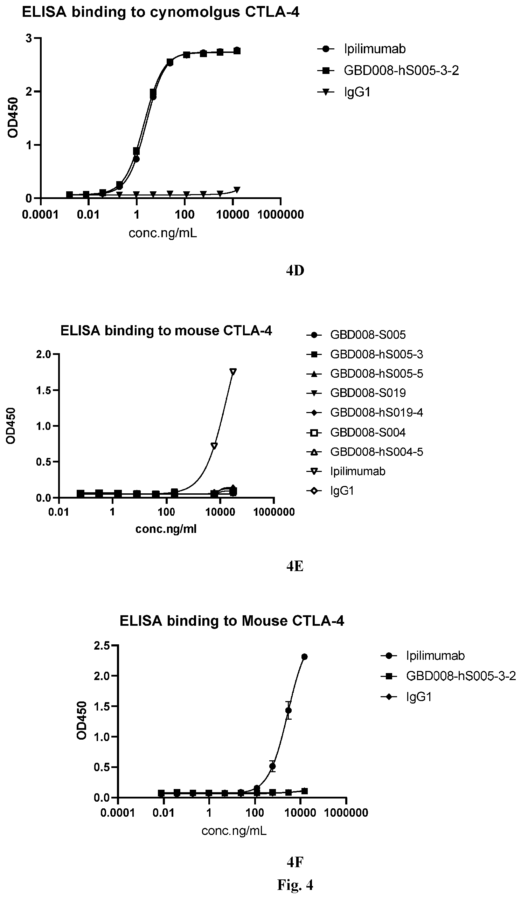

The plate was coated with 1µg/ml human CTLA4 (Acro Biosystem, Catlog #CT4-H52H9), cynomolgus monkey CTLA4 (Acro Biosystem, Catlog #Ct4-C82E5) or mouse CTLA4 (Acro Biosystem, Catlog #CT4-M52H5) at 4°C overnight. The plate was then blocked with blocking buffer at room temperature. After that, a gradient dilution series of anti-CTLA4 antibody was added and the plate was allowed to react at room temperature for 1 hour. Goat anti-human IgG Fc (HRP) (Abcam, Catlog #ab97225) was added and the plate was allowed to react at room temperature for 1 hour. After addition of the colorimetric solution, the reaction was terminated and the absorbance was read at a wavelength of 450 nm using an EnVision 2105 multi-mode plate reader (PerkinElmer). The results are shown in Figures 4A-C.

The absorbance data in Figures 4A-F show that GBD008-S004, GBD008-hS005, GBD008-S019, humanized GBD008-hS005-3, GBD008-hS005-3-2, GBD008-hS005-5, GBD008-hS019-4 and GBD008-hS004-5 antibodies all bind to human CTLA4 and cynomolgus monkey CTLA4, but not to mouse CTLA4.

### Example 7. Assay on ADCC killing by anti-CTLA4 antibodies

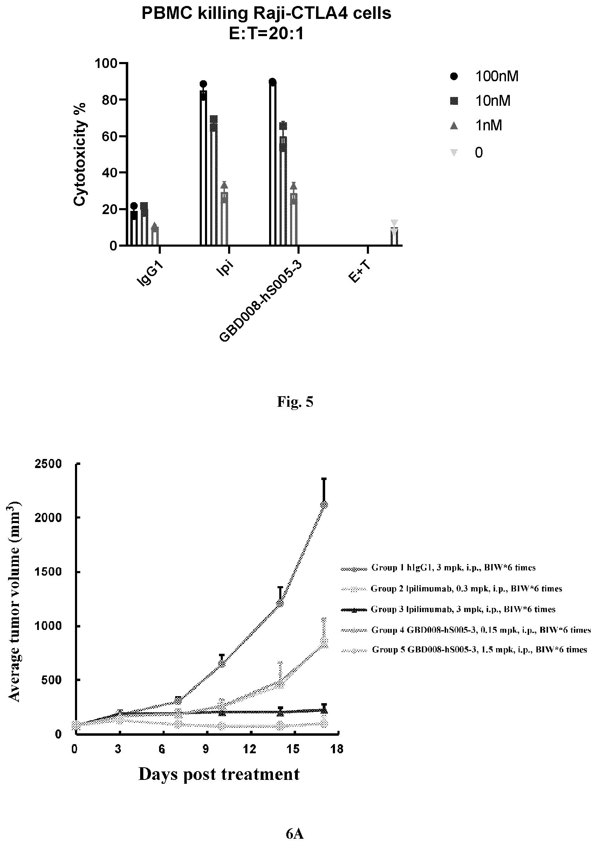

Cytotoxic activity was assessed using FACS analysis. The effector human peripheral blood mononuclear cells were obtained from individual human donors (Milestone^{®} Biotechnologies) and incubated overnight with 10 ng/ml hIL-2 (PeproTech, 200-02). Target Raji cells expressing full-length human CTLA4 were mixed with 3 ml of assay buffer from the EuTDA kit and 5 µl of DELFIA BATDA reagent (PerkinElmer, Catlog #AD16), labelled at 37°C for 20 min, washed twice with 10% FBS (Gibco, Cat. #10099-141) in RPMI 1640 medium (Gibco, Catlog #A10491-01), and seeded in a 96-well round-bottom plate at an effector-to-target cell ratio of 20:1. Serial dilutions of anti-CTLA4 mAb were added to the designated rows of the assay plate. After incubation at 37°C for 2 hours, the plate was centrifuged, and then 20 µl of the supernatant was pipetted and incubated with 200 µl of europium solution at room temperature for 10 minutes. The plate was then read on an EnVision 2105 multimode plate reader (PerkinElmer). The results are shown in Figure 5.

The results demonstrate that the anti-CTLA4 antibodies of the present disclosure potently induce target cell lysis and death.

### Example 8. In vivo antitumor efficacy of anti-CTLA4 antibodies in the MC38 colorectal tumor model

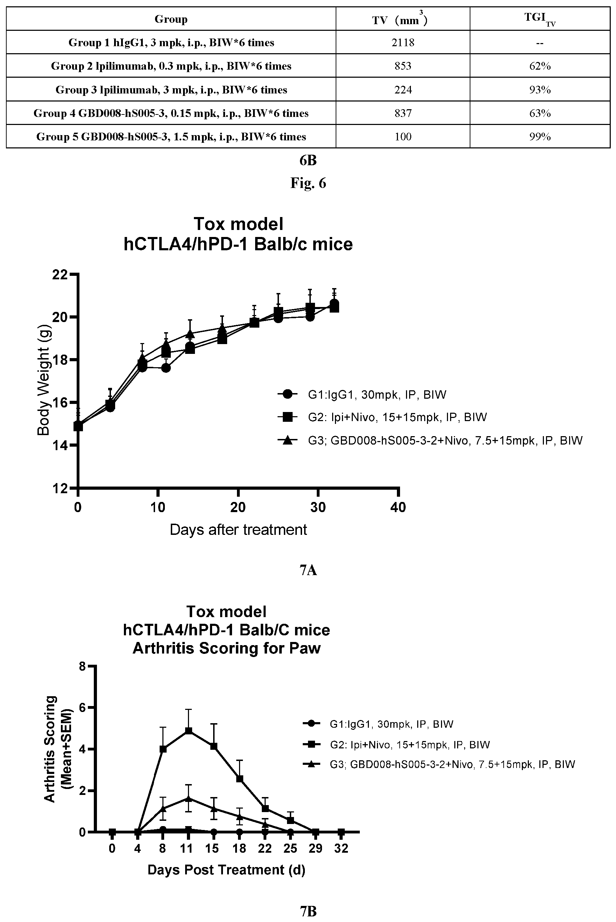

MC38 (NTCC-MC38) C57BL/6 human CTLA4 knock-in mice were subcutaneously inoculated with colon cancer cells (n=6 per group, female, 7-8 weeks old). When tumors were formed (about 80 mm³), two doses of Ipilimumab (0.3 mg/kg and 3 mg/kg) and two doses of the anti-CTLA4 antibody of the present disclosure (0.15 mg/kg and 1.5 mg/kg) were administered via intraperitoneal injection. Treatment was given twice a week for three weeks. Tumor growth was monitored every three days and reported as the average tumor volume. The results are shown in Figure 6.

As can be seen from Figure 6, the average tumor volume of mice treated with the anti-CTLA4 antibody of the present disclosure was significantly smaller than that of the IgG1 control. In addition, at the end of the experiment, for the 0.15 mpk dose, the average tumor volume of the mice was reduced by 62%, and for the 1.5 mpk dose, the average tumor volume of the mice was reduced by up to 99%.

### Example 9. Toxicity of anti-CTLA4 antibodies in the hCTLA 4/hPD1 Bal b/C mouse model (arthritis)

Human CTLA4 /hPD1 knock-in Bal b/C mice (n=8 per group, female, 4 weeks old) were intraperitoneally injected with IgG1 (30 mg/kg), Ipilimumab in combinaion with Nivolumab (15 mg/kg + 15 mg/kg), GBD008-hS005-3-2 in combinaion with Nivolumab (7.5 mg/kg + 15 mg/kg), respectively. Treatment was given twice a week until the arthritis (paw and joint inflammation) of the mice disappeared. The body weight of the mice was monitored and the arthritis of the mice was scored every three or four days. The results are shown in Figures 7A-C.

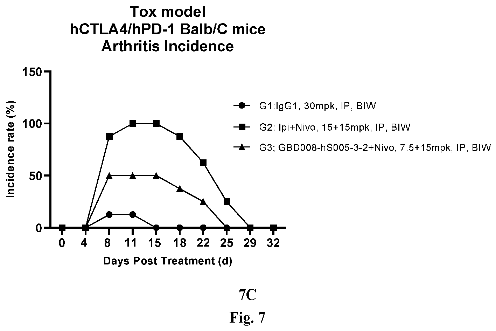

As can be seen from Figure 7B, the arthritis of mice treated with the anti-CTLA4 antibody (GBD008-hS005-3-2) of the present disclosure in combination with Nivolumab was significantly less than that of the IgG1 control and the Ipilimumab and Nivolumab co-administration group. In addition, as can be seen from Figure 7C, the incidence of arthritis in mice treated with GBD008-hS005-3-2 in combination with Nivolumab was also significantly less than that of the IgG1 control and the Ipilimumab and Nivolumab co-administration group.

The above Examples have demonstrated that the anti-CTLA4 antibodies of the present disclosure inhibit the binding of CD80/CD86 to CTLA4 to varying degrees, and also exhibit a potent ADCC response. The anti-CTLA4 antibodies have varying degrees of IL 2 response rates by blocking CD80/86 to varying degrees and retaining the Fc function of the antibodies. In addition, the anti-CTLA4 antibodies of the present disclosure exhibit excellent in vivo anti-tumor efficacy and insignificant toxicity in a mouse model.

Although various embodiments of the present invention have been described above, it should be understood that they are provided by way of illustration only and should not be construed to limit the invention. Various changes and modifications may be made without departing from the spirit and scope of the invention, and these changes and modifications will fall within the scope of the invention as claimed. The scope of the present invention as claimed is defined by the appended claims and their equivalents.

## Claims

1. An antibody or antigen-binding fragment thereof specifically binding to CTLA4, wherein the antibody comprises a heavy chain variable region, wherein the heavy chain variable region comprises CDR1, CDR2 and CDR3, wherein the CDR1, CDR2 and CDR3 comprise an amino acid sequence that is at least 70%, 75%, 80%, 85%, 88%, 90%, 92%, 95%, 97%, 98%, 99% or 100% identical to:
(1) SEQ ID NO: 1, SEQ ID NO: 2 and SEQ ID NO: 3, respectively;
(2) SEQ ID NO: 13, SEQ ID NO: 14 and SEQ ID NO: 15, respectively; or
(3) SEQ ID NO: 22, SEQ ID NO: 23 and SEQ ID NO: 24, respectively.

2. The antibody or antigen-binding fragment thereof according to claim 1, wherein the heavy chain variable region comprises CDR1, CDR2 and CDR3, wherein the CDR1 comprises or consists of the sequence as set forth in SEQ ID NO: 1, the CDR2 comprises or consists of the sequence as set forth in SEQ ID NO: 2, and the CDR3 comprises or consists of the sequence as set forth in SEQ ID NO: 3.

3. The antibody or antigen-binding fragment thereof according to claim 1 or 2, wherein the heavy chain variable region comprises an amino acid sequence that is at least 70%, 75%, 80%, 85%, 88%, 90%, 92%, 95%, 97%, 98%, 99% or 100% identical to SEQ ID NO: 4, SEQ ID NO: 7, SEQ ID NO: 10 or SEQ ID NO: 31.

4. The antibody or antigen-binding fragment thereof according to claim 3, wherein the heavy chain variable region comprises SEQ ID NO: 4 or consists of SEQ ID NO: 4.

5. The antibody or antigen-binding fragment thereof according to claim 4, wherein the heavy chain comprises an amino acid sequence that is at least 70%, 75%, 80%, 85%, 88%, 90%, 92%, 95%, 97%, 98%, 99% or 100% identical to SEQ ID NO: 5.

6. The antibody or antigen-binding fragment thereof according to claim 3, wherein the heavy chain variable region comprises or consists of SEQ ID NO: 7.

7. The antibody or antigen-binding fragment thereof according to claim 6, wherein the heavy chain comprises an amino acid sequence that is at least 70%, 75%, 80%, 85%, 88%, 90%, 92 %, 95%, 97%, 98%, 99% or 100% identical to SEQ ID NO: 8.

8. The antibody or antigen-binding fragment thereof according to claim 3, wherein the heavy chain variable region comprises or consists of SEQ ID NO: 10.

9. The antibody or antigen-binding fragment thereof according to claim 8, wherein the heavy chain comprises an amino acid sequence that is at least 70%, 75%, 80%, 85%, 88%, 90%, 92%, 95%, 97%, 98%, 99% or 100% identical to SEQ ID NO: 11.

10. The antibody or antigen-binding fragment thereof according to claim 3, wherein the heavy chain variable region comprises or consists of SEQ ID NO: 31.

11. The antibody or antigen-binding fragment thereof according to claim 10, wherein the heavy chain comprises an amino acid sequence that is at least 70%, 75%, 80%, 85%, 88%, 90%, 92%, 95%, 97%, 98%, 99% or 100% identical to SEQ ID NO: 32.

12. The antibody or antigen-binding fragment thereof according to claim 1, wherein the heavy chain variable region comprises CDR1, CDR2 and CDR3, wherein the CDR1 comprises or consists of the sequence as set forth in SEQ ID NO: 13, the CDR2 comprises or consists of the sequence as set forth in SEQ ID NO: 14, and the CDR3 comprises or consists of the sequence as set forth in SEQ ID NO: 15.

13. The antibody or antigen-binding fragment thereof according to claim 12, wherein the heavy chain variable region comprises an amino acid sequence that is at least 70%, 75%, 80%, 85%, 88%, 90%, 92%, 95%, 97%, 98%, 99% or 100% identical to SEQ ID NO: 16 or SEQ ID NO: 19.

14. The antibody or antigen-binding fragment thereof according to claim 13, wherein the heavy chain variable region comprises or consists of SEQ ID NO: 16.

15. The antibody or antigen-binding fragment thereof according to claim 14, wherein the heavy chain comprises an amino acid sequence that is at least 70%, 75%, 80%, 85%, 88%, 90%, 92%, 95%, 97%, 98%, 99% or 100% identical to SEQ ID NO: 17.

16. The antibody or antigen-binding fragment thereof according to claim 13, wherein the heavy chain variable region comprises or consists of SEQ ID NO: 19.

17. The antibody or antigen-binding fragment thereof according to claim 14, wherein the heavy chain comprises an amino acid sequence that is at least 70%, 75%, 80%, 85%, 88%, 90%, 92%, 95%, 97%, 98%, 99% or 100% identical to SEQ ID NO: 20.

18. The antibody or antigen-binding fragment thereof according to claim 1, wherein the heavy chain variable region comprises CDR1, CDR2 and CDR3, wherein the CDR1 comprises or consists of the sequence as set forth in SEQ ID NO: 22, the CDR2 comprises or consists of the sequence as set forth in SEQ ID NO: 23, and the CDR3 comprises or consists of the sequence as set forth in SEQ ID NO: 24.

19. The antibody or antigen-binding fragment thereof according to claim 18, wherein the heavy chain variable region comprises an amino acid sequence that is at least 70%, 75%, 80%, 85%, 88%, 90%, 92%, 95%, 97%, 98%, 99% or 100% identical to SEQ ID NO: 25 or SEQ ID NO: 28.

20. The antibody or antigen-binding fragment thereof according to claim 19, wherein the heavy chain variable region comprises or consists of SEQ ID NO: 25.

21. The antibody or antigen-binding fragment thereof according to claim 20, wherein the heavy chain comprises an amino acid sequence that is at least 70%, 75%, 80%, 85%, 88%, 90%, 92%, 95%, 97%, 98%, 99% or 100% identical to SEQ ID NO: 26.

22. The antibody or antigen-binding fragment thereof according to claim 19, wherein the heavy chain variable region comprises or consists of SEQ ID NO: 28.

23. The antibody or antigen-binding fragment thereof according to claim 22, wherein the heavy chain comprises an amino acid sequence that is at least 70%, 75%, 80%, 85%, 88%, 90%, 92%, 95%, 97%, 98%, 99% or 100% identical to SEQ ID NO: 29.

24. The antibody or antigen-binding fragment thereof according to any one of claims 1-23, wherein the antibody or antigen-binding fragment thereof is selected from an intact antibody, a single-chain antibody (scFv), a heavy chain antibody, a single domain antibody, a Fab fragment, a Fab' fragment, a F(ab')2, a Fv, a Fd fragment and a bispecific antibody (BsAb).

25. The antibody or antigen-binding fragment thereof according to any one of claims 1 to 24, wherein the antibody or antigen-binding fragment thereof is a heavy chain-only antibody.

26. The antibody or antigen-binding fragment thereof according to any one of claims 1 to 25, wherein the antibody or antigen-binding fragment thereof comprises or consists of two heavy chains.

27. The antibody or antigen-binding fragment thereof according to any one of claims 1-24, wherein the antibody or antigen-binding fragment thereof is a single domain antibody.

28. The antibody or antigen-binding fragment thereof according to any one of claims 1 to 27, wherein the antibody is a human antibody, a humanized antibody or a chimeric antibody.

29. The antibody according to any one of claims 1-28, wherein the antibody or antigen-binding fragment thereof exhibits significant antibody-dependent cell-mediated cytotoxicity (ADCC).

30. A pharmaceutical composition comprising a therapeutically effective amount of the antibody or antigen-binding fragment thereof according to any one of claims 1 to 19 and a pharmaceutically acceptable carrier.

31. An isolated nucleic acid molecule encoding the antibody or antigen-binding fragment thereof according to any one of claims 1-29.

32. A nucleic acid molecule according to claim 31, wherein the nucleic acid molecule comprises a nucleotide sequence that is at least 70%, 75%, 80%, 85%, 88%, 90%, 92%, 95%, 97%, 98%, 99% or 100% identical to SEQ ID NO: 6, SEQ ID NO: 9, SEQ ID NO: 12, SEQ ID NO: 18, SEQ ID NO: 21, SEQ ID NO: 27, SEQ ID NO: 30 or SEQ ID NO: 33.

33. An expression vector comprising the nucleic acid molecule according to claim 31 or 32.

34. A host cell expressing the antibody or antigen-binding fragment thereof according to any one of claims 1 to 29, and/or comprising the nucleic acid molecule according to claim 31 or 32 or the expression vector according to claim 33.

35. A method for treating a disease or condition in a subject in need thereof, the method comprising administering the antibody or antigen-binding fragment thereof according to any one of claims 1-29 or the pharmaceutical composition of claim 30 in a therapeutically effective amount, wherein the disease or condition is cancer or an autoimmune disease.

36. The method of claim 35, wherein the cancer is selected from the group consisting of colorectal cancer, colon cancer, renal cell carcinoma, breast cancer, epithelial squamous cell carcinoma, melanoma, myeloma, gastric cancer, brain cancer, lung cancer, pancreatic cancer, cervical cancer, ovarian cancer, liver cancer, bladder cancer, prostate cancer, testicular cancer, thyroid cancer, head and neck cancer, leukemia, and lymphoma.

37. The method of claim 35 or 36, wherein the antibody or antigen-binding fragment thereof or the pharmaceutical composition is administered in combination with one or more additional chemotherapeutic agents, radiotherapeutic agents, cytokines or other antibodies.

38. Use of the antibody or antigen-binding fragment thereof according to any one of claims 1 to 29 or the pharmaceutical composition according to claim 30 in the preparation of a medicament for treating a disease or condition, wherein the disease or condition is cancer or an autoimmune disease.

39. The use according to claim 38, wherein the cancer is selected from the group consisting of colorectal cancer, colon cancer, renal cell carcinoma, breast cancer, epithelial squamous cell carcinoma, melanoma, myeloma, gastric cancer, brain cancer, lung cancer, pancreatic cancer, cervical cancer, ovarian cancer, liver cancer, bladder cancer, prostate cancer, testicular cancer, thyroid cancer, head and neck cancer, leukemia and lymphoma.

40. The use according to claim 38 or 39, wherein the antibody or antigen-binding fragment thereof or the pharmaceutical composition is for administration in combination with one or more additional chemotherapeutic agents, radiotherapeutic agents, cytokines or other antibodies.

41. A kit comprising
a) the antibody or antigen-binding fragment thereof according to any one of claims 1 to 29 or the pharmaceutical composition of claim 30; and
b) instructions for use.
